# EUROPEAN PATENT SPECIFICATION

(11) **EP 3 559 230 B1**
(45) Date of publication and mention of the grant of the patent: **06.09.2023**
(21) Application number: 17832436.4
(22) Date of filing: 20.12.2017
(51) Int. Cl.: C12N 15/10, C12N 15/90

(54) **METHODS FOR INCREASING THE EFFICIENCY OF HOMOLOGY DIRECTED REPAIR (HDR) IN THE CELLULAR GENOME**
VERFAHREN ZUR VERBESSERUNG DER EFFIZIENZ VON HOMOLOGIE-GERICHTETER REPARATUR (HDR) IM ZELLULÄREN GENOM
MÉTHODES POUR AUGMENTER L'EFFICACITÉ DE LA RÉPARATION DIRIGÉE PAR L'HOMOLOGIE (HDR) DANS LE GÉNOME CELLULAIRE

(30) Priority: 20.12.2016 US 201662437042 P
(43) Date of publication of application: 30.10.2019
(60) Divisional application: 23188899.1
(73) Proprietor: Bristol-Myers Squibb Company, Princeton, NJ 08543 (US)
(72) Inventor: FEDER, John, Nathan, Princeton, New Jersey 08543 (US); GUO, Qi, Princeton, New Jersey 08543 (US); MINTIER, Gabriel ,Allen, Princeton, New Jersey 08543 (US)
(74) Representative: Mewburn Ellis LLP
(86) International application number: PCT/US2017/067569
(87) International publication number: WO 2018/119060

(56) References cited:
- WO-A1-2016/182959
- WO-A2-2016/154579
- CN-A- 105 734 032
- US-A1- 2011 129 898
- YUICHIRO MIYAOKA ET AL: "Systematic quantification of HDR and NHEJ reveals effects of locus, nuclease, and cell type on genome-editing", SCIENTIFIC REPORTS, vol. 6, no. 1, 31 March 2016 (2016-03-31), XP055460124, DOI: 10.1038/srep23549 cited in the application
- DOMINIK PAQUET ET AL: "Efficient introduction of specific homozygous and heterozygous mutations using CRISPR/Cas9", NATURE, vol. 533, no. 7601, 27 April 2016 (2016-04-27), pages 125-129, XP055380981, GB ISSN: 0028-0836, DOI: 10.1038/nature17664 cited in the application
- L. YANG ET AL: "Optimization of scarless human stem cell genome editing", NUCLEIC ACIDS RESEARCH, vol. 41, no. 19, 31 July 2013 (2013-07-31) , pages 9049-9061, XP055113989, ISSN: 0305-1048, DOI: 10.1093/nar/gkt555 cited in the application
- TAKESHI MARUYAMA ET AL: "Increasing the efficiency of precise genome editing with CRISPR-Cas9 by inhibition of nonhomologous end joining", NATURE BIOTECHNOLOGY, vol. 33, no. 5, 20 April 2015 (2015-04-20) , pages 538-542, XP055290186, ISSN: 1087-0156, DOI: 10.1038/nbt.3190 cited in the application
- YANNICK DOYON ET AL: "Transient cold shock enhances zinc-finger nuclease-mediated gene disruption", NATURE METHODS, vol. 7, no. 6, 2 May 2010 (2010-05-02), pages 459-460, XP055460134, ISSN: 1548-7091, DOI: 10.1038/nmeth.1456 cited in the application
- Q. GUO ET AL: "'Cold shock' increases the frequency of homology directed repair gene editing in induced pluripotent stem cells", SCIENTIFIC REPORTS, vol. 8, no. 1, 1 February 2018 (2018-02-01), XP055459756, DOI: 10.1038/s41598-018-20358-5
- HOCKEMEYER DIRK ET AL: "Induced Pluripotent Stem Cells Meet Genome Editing", CELL STEM CELL, ELSEVIER, CELL PRESS, AMSTERDAM, NL, vol. 18, no. 5, 5 May 2016 (2016-05-05), pages 573-586, XP029530872, ISSN: 1934-5909, DOI: 10.1016/J.STEM.2016.04.013 cited in the application
- JANG YOON-YOUNG ET AL: "Gene correction in patient-specific iPSCs for therapy development and disease modeling", HUMAN GENETICS, SPRINGER, BERLIN, DE, vol. 135, no. 9, 2 June 2016 (2016-06-02), pages 1041-1058, XP036039650, ISSN: 0340-6717, DOI: 10.1007/S00439-016-1691-5 [retrieved on 2016-06-02] cited in the application

## Description

### BACKGROUND OF THE INVENTION

Various methods for DNA-targeted cleavage of genomic sequences have been described in the art. Such targeted cleavage events can be used to induce targeted mutagenesis, induce targeted deletions of cellular DNA sequences, and facilitate targeted recombination at a predetermined chromosomal locus. These methods often involve the use of engineered cleavage systems to induce a double strand break (DSB) or a nick in a target DNA sequence such that repair of the break by an error born process such as non-homologous end joining (NHEJ) or homology directed repair (HDR) can result in the inactivation of a gene or the insertion of an exogenous sequence of interest. Cleavage can occur through the use of specific nucleases such as engineered zinc finger nucleases (ZFN), transcription-activator like effector nucleases (TALENs), using the CRISPR/Cas system with an engineered single guide RNA (sgRNA) to guide specific cleavage. WO 2016/182959 describes CRISPR/Cas-mediated gene editing in stem cells comprising transiently treating the cells with a stem cell viability enhancer prior to and/or after contacting the cells with one or more CRISPR/Cas9 components.

The efficiency of genomic modification at a specific target location through the HDR process is relatively low in cells. Thus, there remains a need for methods for increasing the efficiency of homology directed repair (HDR) in the cellular genome.

### SUMMARY OF THE INVENTION

In certain embodiments, the present invention provides a method for increasing the efficiency of homology directed repair (HDR) in the genome of a cell, comprising: (a) introducing into the cell: (i) a nuclease; and (ii) a donor nucleic acid which comprises a modification sequence to be inserted into the genome, wherein the donor nucleic acid contains symmetrical homology arms and is complementary to the DNA strand in the genome which is cleaved by the nuclease; and (b) subjecting the cell to a temperature shift from 37 °C to a lower temperature which is between 28 °C and 35 °C; wherein the nuclease cleaves the genome at a cleavage site in the cell, and the donor nucleic acid directs the repair of the genome sequence with the modification sequence through an increased rate of HDR; wherein the method does not encompass a process for modifying the germ line genetic identity of human beings. For example, the rate of homology directed repair (HDR) is increased by at least 1.5 fold. Optionally, the rate of homology directed repair (HDR) is increased by at least 2 fold.

Optionally, the lower temperature is between 30 °C and 33 °C. For example, the cell is grown at the lower temperature for at least 24 hours or at least 48 hours, such as between 1 and 5 days (1 day, 2 days, 3 days, 4 days or 5 days). Optionally, the cell is grown at 37 °C after the temperature shift.

In certain aspects, the cell is a eukaryotic cell, such as a mammalian cell. In a specific embodiment, the cell is a stem cell such as an induced pluripotent stem cell (iPSC). In another specific embodiment, the cell is a primary cell.

In certain aspects, the nuclease used in the present invention include all DNA sequence specific endonucleases or RNA guide DNA endonucleases. Optionally, the nuclease is a CRISPR nuclease selected from a Cas nuclease or a Cpf1 nuclease. For example, the nuclease is a Cas9 nuclease. To illustrate, the CRISPR nuclease (e.g., Cas9) is introduced into the cell along with a sgRNA either in a DNA format (e.g., a DNA encoding the Cas9 nuclease and a sgRNA) or an RNA format (e.g., a sgRNA/Cas9 RNP or sgRNA/Cas9 mRNAs). Optionally, the sgRNA is synthetic and chemically modified.

In certain aspects, the nuclease used in the present invention is a zinc finger nuclease (ZFN). In certain other aspects, the nuclease used in the present invention is a TALE nuclease (TALEN).

In certain embodiments, an isolated cell produced by the above-described method is described herein.

In certain embodiments, a pharmaceutical composition comprising an isolated cell produced by the above-described method is described.

In certain embodiments, the present invention provides a cell for use in a therapeutic method of providing a protein of interest to a subject in need thereof, comprising: (a) introducing a donor nucleic acid encoding a protein of interest into a cell according to the above-described method; and (b) introducing the cell into a subject, such that the protein of interest is expressed in the subject; wherein the method does not encompass a process for modifying the germ line genetic identity of human beings.

In certain embodiments, a method for increasing the efficiency of homology directed repair (HDR) in the genome of a cell is described herein, the method comprising introducing into the cell: (i) a nuclease; and (ii) a donor nucleic acid which contains symmetrical homology arms, is complementary to the DNA strand in the genome which is cleaved by the nuclease, and comprises a modification sequence to be inserted into the genome at a distance greater than 10 base pairs away from the cleavage site, wherein the nuclease cleaves the genome at the cleavage site in the cell, and the donor nucleic acid directs the repair of the genome sequence with the modification sequence through an increased rate of HDR. For example, the rate of homology directed repair (HDR) is increased by at least 1.5 fold or at least 2 fold. Optionally, such method further comprises subjecting the cell to a temperature shift from 37 °C to a lower temperature (e.g., between 28 °C and 35 °C or between 30 °C and 33 °C). For example, the cell is grown at the lower temperature for at least 24 hours or at least 48 hours, such as between 1 and 5 days (1 day, 2 days, 3 days, 4 days or 5 days). Optionally, the cell is grown at 37 °C after the temperature shift. In certain aspects, the cell is a eukaryotic cell, such as a mammalian cell. In a specific embodiment, the cell is a stem cell such as an induced pluripotent stem cell (iPSC). In another specific embodiment, the cell is a primary cell. In certain aspects, the nuclease used in the present invention is a CRISPR nuclease selected from a Cas nuclease or a Cpf1 nuclease. For example, the nuclease is a Cas9 nuclease. To illustrate, the CRISPR nuclease (e.g., Cas9) is introduced into the cell along with a sgRNA either in a DNA format (e.g., a DNA encoding the Cas9 nuclease and a sgRNA) or an RNA format (e.g., a sgRNA/Cas9 RNP or sgRNA/Cas9 mRNAs). In certain aspects, the nuclease used in the present invention is a zinc finger nuclease (ZFN). In certain other aspects, the nuclease used in the present invention is a TALE nuclease (TALEN).

### BRIEF DESCRIPTION OF THE DRAWINGS

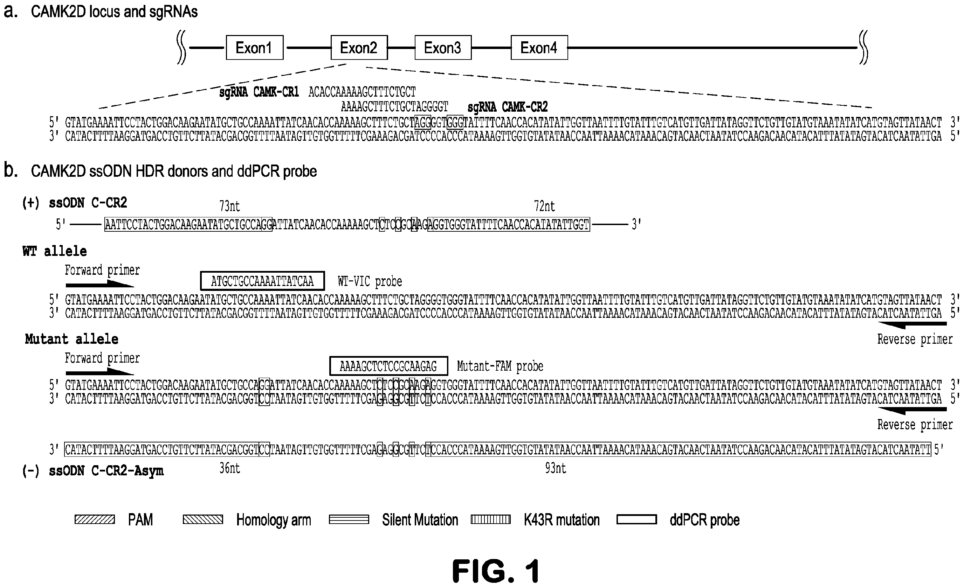

Figures 1a-1b show the single-stranded oligonucleotide (ssODN) donor design, droplet digital PCR probes and primer designs for gene editing and mutation detection at CAMK2D locus. (a) Two guide RNAs (CAMK-CR1 and CAMK-CR2) were designed to specifically target CAMK2D Exon2, CAMK-CR1 and CAMK-CR2 overlap by 14 nucleotides and are designed to cleave the DNA to introduce the same sequence alteration by HDR. (b) Two ssODN HDR donors (C-CR2 and C-CR2-Asym) were designed to introduce a kinase dead K43R mutation (AAA to AGG) and four silent mutations into Exon2 of the CAMK2D locus. The ssODN donor C-CR2 is a (+) strand HDR donor which is complementary to the guide RNA targeted cleavage strand with balanced homology arms around each side of the intended mutations (5'-73nt and 3'-72nt, respectively). C-CR2-Asym is a (-) strand HDR donor which is complementary to the guide RNA non-targeted strand with homology arms that differ in length (5'-93nt and 3'-36nt, respectively). To prevent subsequent re-cleavage, both donor oligo C-CR2 and C-CR2-Asym introduces three silent mutations within the guide CAMK-CR1 recognition site and one silent mutation within the PAM site. C-CR2 and C-CR2-Asym introduces four silent mutations within the guide CAMK-CR2 recognition site. A pair of primers and allele-specific probes conjugated with Vic or Fam fluorophores were also designed to detect separately the unaltered wild type alleles and mutated sequence conversion events. The forward primer was designed to anneal within the donor sequence while the reverse primer was designed to anneal outside of the donor sequence to ensure the proper locus was amplified.

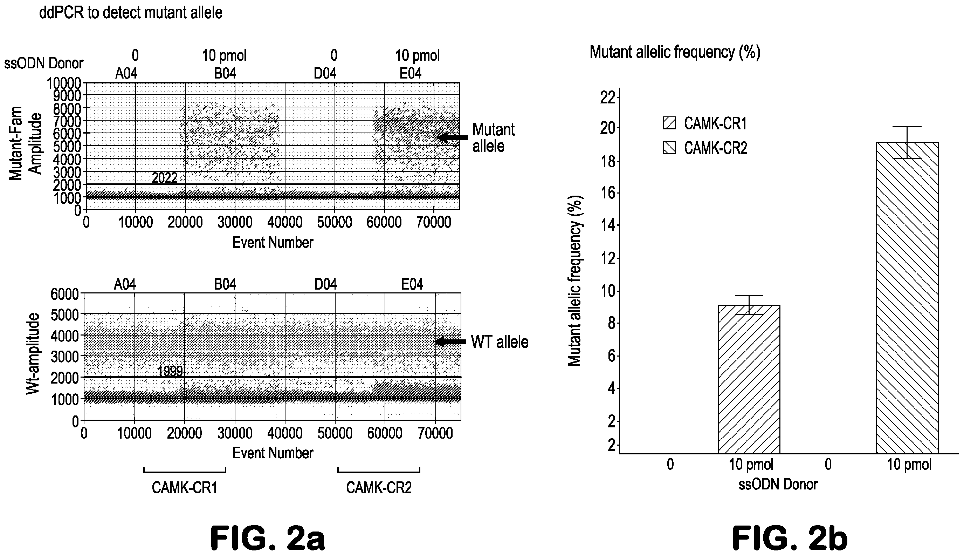

Figures 2a-2c show an optimized method for co-delivery of a single-stranded oligonucleotide (ssODN) donor and sgRNA/Cas9 mRNA to perform HDR at the CAMK2D locus in mc-iPSCs. sgRNA CAMK-CR1 or CAMK-CR2, Cas9 mRNA and ssODN donor C-CR2 were co-transfected into mc-iPSCs using the EditPro^{™} RNA transfection reagent. (a) The percent wild type and mutant alleles from the transfected cells were detected by droplet digital PCR (ddPCR) using a wild type alleles specific fluorescence probes(VIC) and mutant alleles specific fluorescence probes (FAM), the fluorescence intensity of each droplet in the sample is plotted versus droplet number. Droplets that have fluorescence intensity above the pink threshold line are counted as positive for the target alleles. The bottom panel (green) represents the droplet of wild type alleles while the top panel (blue) represents the alleles that have undergone HDR. The data presented is from one representative experiment using the two sgRNAs at the best concentration of ssOND, 10 pmole. (b) Quantification of mutant allelic frequency. Data presented as mean ± SEM from four independent experiments. sgRNA CAMK-CR2 consistently produced HDR at greater than 15% of the total allele. (c) Quantification of the intended nucleotide changes by next generation sequencing using the same gRNA and donor used for the ddPCR experiments. Each bar represents one of the six nucleotide changes included on the donor oligo. The data indicates that complete sequence conversion occurred across the targeted region and at frequencies that were in agreement with the ddPCR results. The two A to G changes not directly measured by ddPCR were also incorporated albeit at lower frequencies compared to those changes that were closer to the CRISPR cleavage site. The data presented is the mean percent intended base alteration at each of their exact genomic coordinates from four independent experiments.

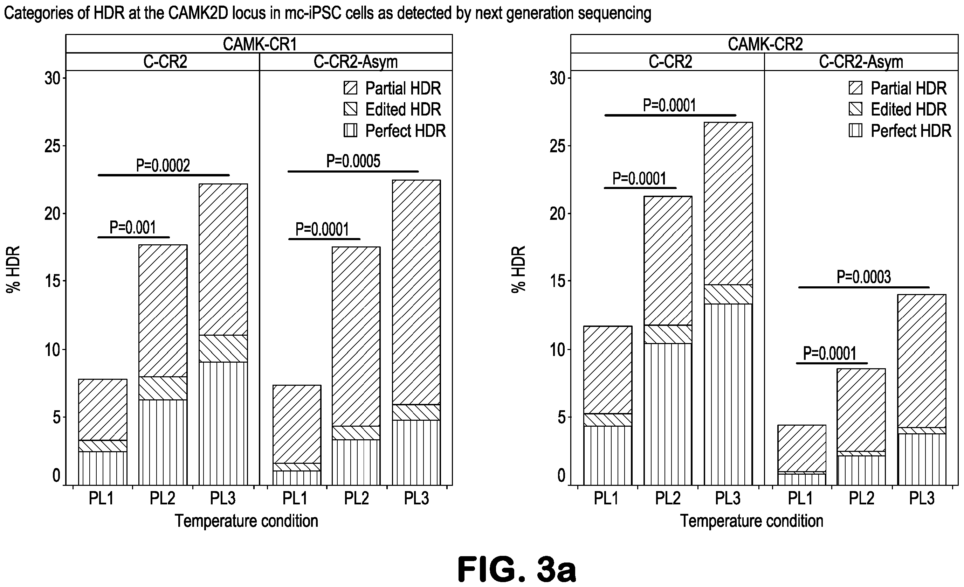

Figures 3a-3b show the effects of 'cold shock' and ssODN HDR donor designs on HDR efficiencies at the CAMK2D locus in mc-iPSCs as determined by NGS. Various amounts of ssODN C-CR2 or C-CR2-Asym were delivered to mc-iPSCs along with Cas9 mRNA and sgRNA CAMK-CR1 or CAMK-CR2 to achieve HDR at the CAMK2D locus. The experiments were carried out at different temperatures over 24 hour intervals as described in "material and methods": PL1: 37 °C-37 °C-37 °C, PL2: 37 °C-32 °C-37 °C, PL3: 37 °C-32 °C-32 °C. (a) HDR events using 10 pmol of ssODN HDR donors for each treatment (CAMK-CR1 with C-TR2 or C-TR2-Asym, CAMK-CR2 with C-TR2 or C-TR2-Asym) were determined by NGS as described in "material and methods". The data presented are the mean percent HDR events (C-CR2: 8 replicates from three independent experiments; C-CR2-Asym: 6 replicates from two independent experiments). The HDR types were categorized into three groups based on the resulting sequence around the region of the intended mutations. Perfect HDR: All intended base changes are present with no re-editing indels. Edited HDR: One or more of the intended base changes are present with re-editing indels present. Partial HDR: Some but not all of the intended base changes with no indels. Data demonstrates that increased HDR can be achieved by `cold shocking' the cells and that the majority of the increase is in the 'Perfect HDR' category. The significance of total HDR efficiencies difference among three temperature conditions for each gRNA and ssODN treatment were analyzed by one way ANOVA (one way ANOVA P<0.0001 for all gRNA and ssODN treatment, P value of follow-up Dunnett's multiple comparison are shown in the figures). HDR from 30 pmol ssODN and no oligo treatment were shown in table 4 (b) Perfect HDR events of each treatment (CAMK-CR1 with C-TR2 or C-TR2-Asym, CAMK-CR2 with C-TR2 or C-TR2-Asym) were plotted to compare perfect HDR frequencies between the two ssODN design. Data presented are the mean percent of perfect HDR events ± SEM (6 biological replicates from two independent experiments), The difference of perfect HDR frequencies between the two ssODN design in each treatment group were evaluated by Student's T-test and p values are shown in the figure. (+) strand ssODN C-CR2 promote more perfect HDR than (-) strand ssODN C-CR2-Asym across all temperature conditions.

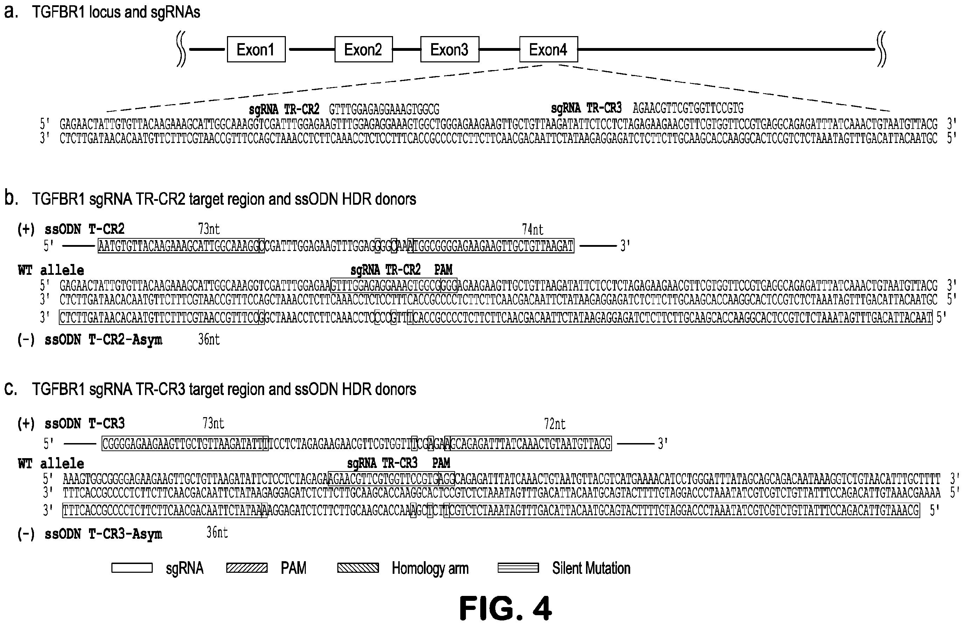

Figures 4a-4c show the guide RNAs and single-stranded oligonucleotide (ssODN) donor designs for gene editing at the TGFBR1 locus. (a) Two guide RNAs TR-CR2 and TR-CR3 were designed to specifically target TGFBR1 Exon 4 and introduce different sequence alteration by HDR. TR-CR3 is 39 nucleotides downstream of TR-CR2. (b) Two ssODN HDR donors (T-CR2 and T-CR2-Asym) were designed to introduce a silent mutation 12 bp upstream of guide RNA TR-CR2 target site, with three silent mutations within the guide RNA recognition sequence to prevent re-editing of the HDR converted sequence. T-CR2 is a (+) strand HDR donor which is complementary to the guide RNA targeted cleavage strand with balanced homology arms around each side of the intended mutations (5'-73nt and 3'-74nt, respectively). T-CR2-Asym is a (-) strand HDR donor which is complementary to the guide RNA non-targeted strand with homology arms that differ in length (5'-93nt and 3'- 36nt, respectively). (c) Two ssODN donors (T-CR3 and T-CR3-Asym) were designed to introduce a known SNP 12 bp upstream of guide RNA TR-CR3 target site, with two silent mutations within the guide RNA recognition sequence and one silent mutation within the TR-CR3 PAM site to prevent re-editing of the HDR converted sequence. T-CR3 is a (+) strand HDR donor which is complementary to the guide RNA targeted strand with balanced length homology arms (5'-73nt and 3'-72nt, respectively) around each side of the intended mutations. T-CR3-Asym is a (-) strand HDR donor which is complementary to the guide RNA non-targeted strand with unbalanced length homology arms (5'-86nt and 3'-36nt, respectively) around each side of the intended mutations.

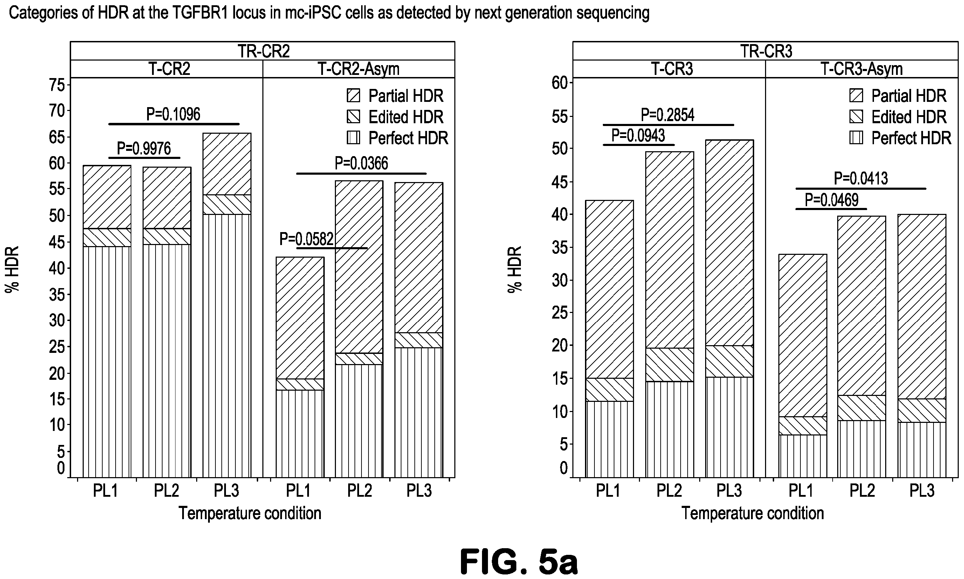

Figures 5a-5b show the effects of 'cold shock' and ssODN HDR donor designs on HDR efficiency at the TGFBR1 locus in mc-iPSCs. ssODN HDR donors and sgRNAs were co-delivered into mc-iPSCs cells along with Cas9 mRNA to achieve HDR at the TGFBR1 locus. Experiments were carried out at different temperatures over 24 hour intervals as described in "material and methods": PL1: 37 °C-37 °C-37 °C, PL2: 37 °C-32 °C-37 °C, PL3: 37 °C-32 °C-32 °C, PL4: 32 °C-32 °C-32 °C. (a) HDR events using 10 pmol of ssODN HDR donors for each treatment (TR-CR2 with T-TR2 or T-TR2-Asym, TR-CR3 with T-TR3 or T-TR3-Asym) were determined by NGS as described in "material and methods". The data presented are the mean percentage HDR events (4 replicates from three independent experiments). The HDR types were categorized into three groups based on the resulting sequence around the region of the intended mutations. Perfect HDR: All intended base changes are present with no re-editing indels. Edited HDR: One or more of the intended base changes are present with re-editing indels. Partial HDR: Some but not all of the intended base changes with no indels. The significance of total HDR efficiencies difference among three temperature conditions for each gRNA and ssODN treatment were analyzed by one way ANOVA (one way ANOVA P>0.05, P value of follow-up Dunnett's multiple comparison are shown in the figures). HDR from 30 pmol ssODN and no oligo treatment were shown in table 5 (b) Perfect HDR events using 10 pmol of ssODN HDR donors for each treatment (TR-CR2 with T-TR2 or T-TR2-Asym, TR-CR3 with T-TR3 or T-TR3-Asym) were plotted to compare perfect HDR frequencies between the two ssODN design. Data presented are the mean percent of perfect HDR events ± SEM (three independent experiments with 4 replicates). The difference of perfect HDR frequencies between the two ssODN design in each treatment group were evaluated by Student's T-test and p values are shown in the figure. Across all temperature conditions, (+) ssODN strand T-CR2 and T-CR3 promote more perfect HDR than (-) ssODN strand T-CR2-Asym and T-CR3-Asym, respectively.

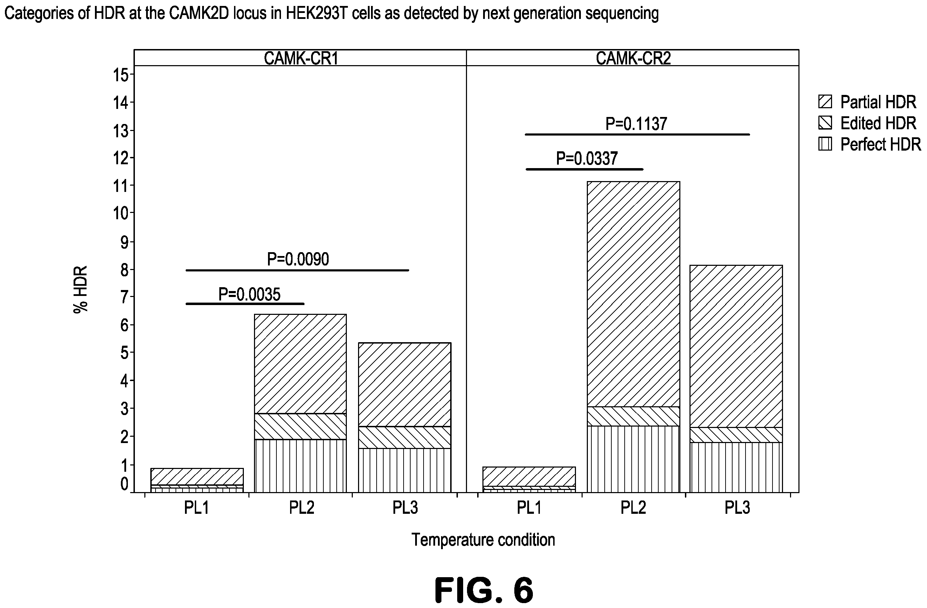

Figure 6 shows that `cold shock' enhances HDR efficiencies at the CAMK2D locus in HEK293T cells. Various amount of ssODN C-CR2 were delivered to HEK293T cells along with Cas9 mRNA and sgRNA CAMK-CR1 or CAMK-CR2 using the same transfection conditions as for mc-iPSC to achieve HDR at the CAMK2D locus. The experiments were carried out at different temperatures over 24 hour intervals as described in "material and methods": PL1: 37°C-37°C-37°C, PL2: 37°C-32°C-37°C, PL3: 37°C-32°C-32°C (a) HDR events using 10 pmol of ssODN HDR donors for each treatment were determined by NGS as described in "material and methods". Data presented as the mean percentage HDR events from three replicates. The HDR types were categorized into three groups based on the resulting sequence around the region of intended mutations. Perfect HDR: All intended base changes occur and no indels. Edited HDR: One or more intended base changes occur, but there are indels. Partial HDR: Some but not all intended base changes occur, and no indels. The significance of total HDR efficiencies difference among three temperature conditions for each gRNA and ssODN treatment were analyzed by one way ANOVA (one way ANOVA: CAMK-CR1 with C-CR2, P=0.0041; CAMK-CR2 with C-CR2, P=0.0469; P value of follow-up Dunnett's multiple comparison are shown in the figures). HDR from 30 pmol ssODN and no oligo treatment were shown in table 7.

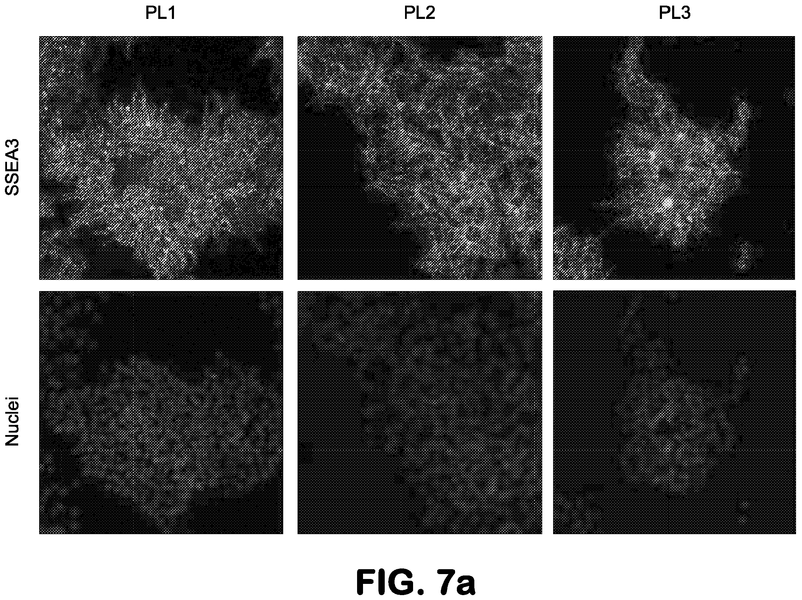

Figures 7a-7c show the expression of pluripotency markers in mc-iPSCs after `cold shock'. Mc-iPSC were grown at different temperatures over 24 hour intervals as described in "supplementary methods": PL1: 37 °C-37 °C-37 °C, PL2: 37 °C-32 °C-37 °C, PL3: 37 °C-32 °C-32 °C. The cells were then stained with pluripotency specific antibodies as described in "supplementary methods": (a) SSEA3 (green), (b) Nanog (green) and (c) OCT4 (green). The cells were also co-stained with Hoechst to label nuclei (blue).

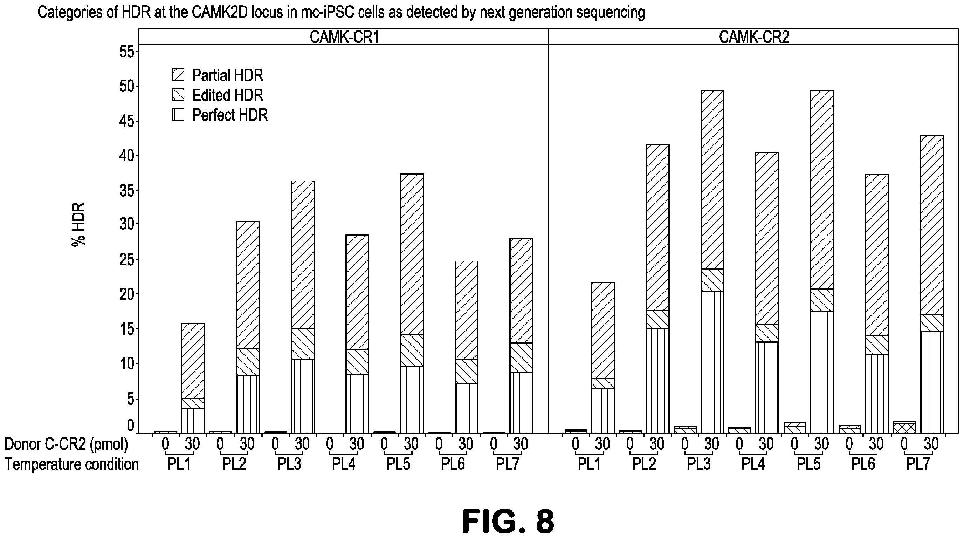

Figure 8 shows that `cold shock' enhances HDR efficiencies at the CAMK2D locus in mc-iPSCs as determined by NGS. 30 pmol of ssODN C-CR2 were delivered to mc-iPSCs along with Cas9 mRNA and sgRNA CAMK-CR1 or CAMK-CR2 to achieve HDR at the CAMK2D locus. The experiments were carried out at different temperatures over 24 hour intervals as described in "material and methods": PL1: 37 °C-37 °C-37 °C, PL2: 37 °C-32 °C-37 °C, PL3: 37 °C-32 °C-32 °C, PL4: 32 °C-30 °C-37 °C, PL5: 37 °C-30 °C-30 °C, PL6: 37 °C-28 °C-37 °C, PL7: 37 °C-28 °C-28 °C. (a) HDR events for each treatment were determined by NGS as described in "material and methods". The data presented are the mean percent HDR events from two replicates. The HDR types were categorized into three groups based on the resulting sequence around the region of the intended mutations. Perfect HDR: All intended base changes are present with no re-editing indels. Edited HDR: One or more of the intended base changes are present with re-editing indels present. Partial HDR: Some but not all of the intended base changes with no indels. The low percentage of Edited HDR and Partial HDR sequences in the no oligo treatment represent background error rates associated with next generation sequencing. No Perfect HDR detected in the no oligo treatments. Data demonstrates that increased HDR can be achieved by `cold shocking' the cells and that the majority of the increase is in the 'Perfect HDR' category.

### DETAILED DESCRIPTION OF THE INVENTION

In certain aspects, the present invention is directed to methods for increasing the efficiency of homology directed repair (HDR) in the genome of a cell, such as by using CRISPR/Cas9 technology; wherein the method does not encompass a process for modifying the germ line genetic identity of human beings. As described in the working examples, Applicants demonstrated that low HDR rates (between 1-20%) can be enhanced two- to ten-fold in cells (e.g., iPSCs and HEK293 cells) by `cold shocking' cells at a lower temperature following transfection. This method also increases the proportion of loci that have undergone complete sequence conversion across the donor sequence, or `perfect HDR', as opposed to partial sequence conversion where nucleotides more distal to the CRISPR cut site are less efficiently incorporated ('partial HDR'). It was also demonstrated in the working examples that the structure of the single-stranded DNA oligo donor can greatly influence the fidelity of HDR, with oligos symmetric with respect to the CRISPR cleavage site and complementary to the target strand being more efficient at directing `perfect HDR' compared to asymmetric non-target strand complementary oligos.

In certain embodiments, the present invention provides a method for increasing the efficiency of homology directed repair (HDR) in the genome of a cell, comprising: (a) introducing into the cell: (i) a nuclease; and (ii) a donor nucleic acid which comprises a modification sequence to be inserted into the genome, wherein the donor nucleic acid contains symmetrical homology arms and is complementary to the DNA strand in the genome which is cleaved by the nuclease; and (b) subjecting the cell to a temperature shift from 37 °C to a lower temperature which is between 28 °C and 35 °C; wherein the nuclease cleaves the genome at a cleavage site in the cell, and the donor nucleic acid directs the repair of the genome sequence with the modification sequence through an increased rate of HDR: wherein the method does not encompass a process for modifying the germ line genetic identity of human beings. For example, the rate of homology directed repair (HDR) is increased by at least 1.5 fold. Optionally, the rate of homology directed repair (HDR) is increased by at least 2 fold.

Optionally, the lower temperature is between 30 °C and 33 °C. For example, the cell is grown at the lower temperature for at least 24 hours or at least 48 hours, such as between 1 and 5 days (1 day, 2 days, 3 days, 4 days or 5 days). Optionally, the cell is grown at 37 °C after the temperature shift.

In certain aspects, the cell is a eukaryotic cell, such as a mammalian cell. In a specific embodiment, the cell is a stem cell such as an induced pluripotent stem cell (iPSC). In another specific embodiment, the cell is a primary cell. In another specific embodiment, the cell is a plant cell.

In certain aspects, the nuclease used in the present invention include all DNA sequence specific endonucleases or RNA guide DNA endonucleases. Optionally, the nuclease is a CRISPR nuclease selected from a Cas nuclease or a Cpf1 nuclease. For example, the nuclease is a Cas9 nuclease. To illustrate, the CRISPR nuclease (e.g., Cas9) is introduced into the cell along with a sgRNA either in a DNA format (e.g., a DNA encoding the Cas9 nuclease and a sgRNA) or an RNA format (e.g., a sgRNA/Cas9 RNP or sgRNA/Cas9 mRNAs). Optionally, the sgRNA is synthetic and chemically modified.

In certain aspects, the nuclease used in the present invention is a zinc finger nuclease (ZFN). In certain other aspects, the nuclease used in the present invention is a TALE nuclease (TALEN).

In certain embodiments, an isolated cell produced by the above-described method is described herein.

In certain embodiments, a pharmaceutical composition comprising an isolated cell produced by the above-described method is described herein.

In certain embodiments, the present invention provides a cell for use in a therapeutic method of providing a protein of interest to a subject in need thereof, comprising: (a) introducing a donor nucleic acid encoding a protein of interest into a cell according to the above-described method; and (b) introducing the cell into a subject, such that the protein of interest is expressed in the subject; wherein the method does not encompass a process for modifying the germ line genetic identity of human beings.

In certain embodiments, a method for increasing the efficiency of homology directed repair (HDR) in the genome of a cell is described herein, the method comprising introducing into the cell: (i) a nuclease; and (ii) a donor nucleic acid which contains symmetrical homology arms, is complementary to the DNA strand in the genome which is cleaved by the nuclease, and comprises a modification sequence to be inserted into the genome at a distance greater than 10 base pairs away from the cleavage site, wherein the nuclease cleaves the genome at the cleavage site in the cell, and the donor nucleic acid directs the repair of the genome sequence with the modification sequence through an increased rate of HDR. For example, the rate of homology directed repair (HDR) is increased by at least 1.5 fold or at least 2 fold. Optionally, such method further comprises subjecting the cell to a temperature shift from 37 °C to a lower temperature (e.g., between 28 °C and 35 °C or between 30 °C and 33 °C). For example, the cell is grown at the lower temperature for at least 24 hours or at least 48 hours, such as between 1 and 5 days (1 day, 2 days, 3 days, 4 days or 5 days).

### I. Definitions

In order that the present disclosure may be more readily understood, certain terms are first defined. As used in this application, except as otherwise expressly provided herein, each of the following terms shall have the meaning set forth below. Additional definitions are set forth throughout the application.

The terms "nucleic acid," "polynucleotide," and "oligonucleotide" are used interchangeably and refer to a deoxyribonucleotide or ribonucleotide polymer, in linear or circular conformation, and in either single- or double-stranded form.

The terms "polypeptide," "peptide" and "protein" are used interchangeably to refer to a polymer of amino acid residues.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

A "TALE DNA binding domain" or "TALE" is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein.

The term "CRISPR/Cas9 system" or "Cas9 system" refers to a system capable of altering a target nucleic acid by one of many DNA repair pathways. In certain embodiments, the Cas9 system described herein promotes repair of a target nucleic acid via an HDR pathway. In some embodiments, a Cas9 system comprises a gRNA molecule and a Cas9 molecule. In some embodiments, a Cas9 system further comprises a second gRNA molecule.

A "Cas9 molecule" or "Cas9 nuclease," as used herein, refers to a Cas9 polypeptide or a nucleic acid encoding a Cas9 polypeptide. A "Cas9 polypeptide" is a polypeptide that can interact with a gRNA molecule and, in concert with the gRNA molecule, localize to a site comprising a target domain and, in certain embodiments, a PAM sequence. Cas9 molecules include both naturally occurring Cas9 molecules, engineered, altered or modified Cas9 molecules, as well as Cas9 polypeptides that differ, e.g., by at least one amino acid residue, from a reference Cas9 sequence, e.g., the naturally occurring Cas9 molecule. The terms "altered, engineered or modified," as used in this context, refer merely to a difference from a reference or naturally occurring Cas9 sequence, and impose no specific process or origin limitations. A Cas9 molecule may be a nuclease (an enzyme that cleaves both strands of a double- stranded nucleic acid) or a nickase (an enzyme that cleaves one strand of a double- stranded nucleic acid).

As used herein, the term "gRNA molecule" or "gRNA" refers to a guide RNA which is capable of targeting a Cas9 molecule to a target nucleic acid. In one embodiment, the term "gRNA molecule" refers to a guide ribonucleic acid. In another embodiment, the term "gRNA molecule" refers to a nucleic acid encoding a gRNA. In one embodiment, a gRNA molecule is non-naturally occurring. In one embodiment, a gRNA molecule is a synthetic gRNA molecule. In anther embodiment, a gRNA molecule is chemically modified.

A "template nucleic acid," "donor nucleic acid," or "donor polynucleotide" refers to a nucleic acid sequence which can be used in conjunction with a nuclease (e.g., a Cas9 molecule) to alter the structure of a target position. In an embodiment, the template nucleic acid is modified to have the some or all of the sequence of the template nucleic acid, typically at or near cleavage site(s). In an embodiment, the template nucleic acid is single stranded. In an alternate embodiment, the template nucleic acid is double stranded. In an embodiment, the template nucleic acid is DNA, e.g., a double stranded DNA. In an alternate embodiment, the template nucleic acid is a single stranded DNA. In an embodiment, the template nucleic acid is an RNA, e.g., a double stranded RNA or a single stranded RNA. In one embodiment, the template nucleic acid is an exogenous nucleic acid sequence. In another embodiment, the template nucleic acid sequence is an endogenous nucleic acid sequence, e.g., an endogenous homologous region. In one embodiment, the template nucleic acid is a single stranded oligonucleotide corresponding to a plus strand of a nucleic acid sequence. In another embodiment, the template nucleic acid is a single stranded oligonucleotide corresponding to a minus strand of a nucleic acid sequence.

"Homology-directed repair" or "HDR" refers to the process of repairing DNA damage in cells using a homologous nucleic acid (e.g., an endogenous homologous sequence, e.g., a sister chromatid, or an exogenous nucleic acid, e.g., a template nucleic acid). Canonical HDR typically acts when there has been significant resection at the double strand break, forming at least one single stranded portion of DNA. In a normal cell, HDR typically involves a series of steps such as recognition of the break, stabilization of the break, resection, stabilization of single stranded DNA, formation of a DNA crossover intermediate, resolution of the crossover intermediate, and ligation.

"Non-homologous end joining" or "NHEJ" refers to ligation mediated repair and/or non-template mediated repair including canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ).

"Recombination" refers to a process of exchange of genetic information between two polynucleotides, including but not limited to, donor capture by non-homologous end joining (NHEJ) and homologous recombination. "Homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule, leading to the transfer of genetic information from the donor to the target. Such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

In the methods of the disclosure, a nuclease as described herein creates a double-stranded break in the target sequence (e.g., cellular chromatin) at a predetermined recognition site, and a "donor" polynucleotide, having homology to the nucleotide sequence in the region of the break, can be introduced into the cell. The presence of the double-stranded break has been shown to facilitate repair of the genome sequence by the donor polynucleotide. The donor polynucleotide may be physically integrated or, alternatively, the donor polynucleotide is used as a template for repair of the break via homologous recombination, resulting in the introduction of all or part of the nucleotide sequence as in the donor into the cellular chromatin. Thus, a first sequence in cellular chromatin can be altered and, in certain embodiments, can be converted into a sequence present in a donor polynucleotide (herein referred to as a "modification sequence"). Thus, the use of the terms "replace" or "replacement" can be understood to represent replacement of one nucleotide sequence by another, and does not necessarily require physical or chemical replacement of one polynucleotide by another.

### II. Nucleases

Methods of the present invention utilize nucleases for cleavage of the genome of a cell such that a template nucleic acid (a transgene) direct repair of the genome sequence in a targeted manner. In certain embodiments, the nucleases are naturally occurring. In other embodiments, the nucleases are non-naturally occurring, e.g., engineered or modified versions of the naturally occurring wildtype nuclease.

Nucleases include, but are not limited to, Cas proteins, DNA sequence specific endonucleases, RNA-guided DNA endonucleases (e.g., Cpf1), restriction endonucleases, meganucleases, homing endonucleases, TAL effector nucleases, and Zinc finger nucleases. Exemplary nucleases include, but are not limited to, Type I, Type II, Type III, Type IV, and Type V endonucleases. For example, the nuclease is a CRISPR nuclease (e.g., a Cas nuclease or a Cpf1 nuclease). In some specific embodiments, the nuclease is Cas9, for example, a Cas9 cloned or derived from a bacteria (e.g., S. pyogenes, S. pneumoniae, S. aureus, or S. thermophilus).

In certain embodiments, the nuclease is the CRISPR/Cas nuclease system. CRISPR (clustered regularly interspaced short palindromic repeats) locus, which encodes RNA components of the system, and the cas (CRISPR-associated) locus, which encodes proteins (Jansen et al., 2002. Mol. Microbiol. 43: 1565-1575; Makarova et al., 2002. Nucleic Acids Res. 30: 482-496; Makarova et al., 2006. Biol. Direct 1: 7; Haft et al., 2005. PLoS Comput. Biol. 1:e60) make up the gene sequences of the CRISPR/Cas nuclease system. CRISPR loci in microbial hosts contain a combination of CRISPR-associated (Cas) genes as well as non-coding RNA elements capable of programming the specificity of the CRISPR-mediated nucleic acid cleavage.

The Type II CRISPR is one of the most well characterized systems and carries out targeted DNA double-strand break in four sequential steps. First, two non-coding RNA, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Wastson-Crick base-pairing between the spacer on the crRNA and the protospacer on the target DNA next to the protospacer adjacent motif (PAM), an additional requirement for target recognition. Finally, Cas9 mediates cleavage of target DNA to create a double-stranded break within the protospacer.

In certain embodiments, Cas protein may be a "functional derivative" of a naturally occurring Cas protein. A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof. Suitable derivatives of a Cas polypeptide or a fragment thereof include but are not limited to mutants, fusions, covalent modifications of Cas protein or a fragment thereof. Cas protein, which includes Cas protein or a fragment thereof, as well as derivatives of Cas protein or a fragment thereof, may be obtainable from a cell or synthesized chemically or by a combination of these two procedures. The cell may be a cell that naturally produces Cas protein, or a cell that naturally produces Cas protein and is genetically engineered to produce the endogenous Cas protein at a higher expression level or to produce a Cas protein from an exogenously introduced nucleic acid, which nucleic acid encodes a Cas that is same or different from the endogenous Cas. In some case, the cell does not naturally produce Cas protein and is genetically engineered to produce a Cas protein.

In other embodiments, the nuclease may be a zinc finger nuclease (ZFN) or a transcription activator-like effector nucleases (TALEN). ZFNs and TALENs comprise heterologous DNA-binding and cleavage domains. These molecules are well known genome editing tools. See, e.g., Gai, et al., Trends Biotechnol. 2013 Jul; 31(7): 397-405.

### III. Host Cells

Any host cell wherein a genomic modification may be used in the present invention. The cell types can be cell lines or natural (e.g., isolated) cells such as, for example, primary cells.

To illustrate, suitable cells include eukaryotic (e.g., animal, plant, mammalian) cells and/or cell lines. Non-limiting examples of such cells or cell lines include COS, CHO (e.g., CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T), and perC6 cells. In certain embodiments, the cell line is a CHO, MDCK or HEK293 cell line. Suitable cells also include stem cells such as, by way of example, non-human embryonic stem cells, induced pluripotent stem cells, hematopoietic stem cells, neuronal stem cells, and mesenchymal stem cells.

### IV. Delivery Methods

The nucleases, nucleic acids encoding these nucleases, template nucleic acids, and compositions comprising the proteins and/or nucleic acids may be delivered in vivo or ex vivo by any suitable means into any cell type.

Nucleases and/or donor constructs as described herein may also be delivered using vectors containing sequences encoding one or more of the ZFN(s), TALEN(s) or CRIPSR/Cas sytems. Any vector systems may be used including, but not limited to, plasmid vectors, retroviral vectors, lentiviral vectors, adenovirus vectors, poxvirus vectors; herpesvirus vectors and adeno-associated virus vectors, etc. See, also, U.S. Pat. Nos. 6,534,261; 6,607,882; 6,824,978; 6,933,113; 6,979,539; 7,013,219; and 7,163, 824. Furthermore, it will be apparent that any of these vectors may comprise one or more of the sequences needed for treatment. Thus, when one or more nucleases and a donor construct are introduced into the cell, the nucleases and/or donor polynucleotide may be carried on the same vector or on different vectors. When multiple vectors are used, each vector may comprise a sequence encoding one or multiple nucleases and/or donor constructs.

Conventional viral and non-viral based gene transfer methods can be used to introduce nucleic acids encoding nucleases and donor constructs in cells (e.g., mammalian cells) and target tissues. Non-viral vector delivery systems include DNA or RNA plasmids, DNA MCs, naked nucleic acid, and nucleic acid complexed with a delivery vehicle such as a liposome or poloxamer. Viral vector delivery systems include DNA and RNA viruses, which have either episomal or integrated genomes after delivery to the cell.

Methods of non-viral delivery of nucleic acids include electroporation, lipofection, microinjection, biolistics, virosomes, liposomes, immunoliposomes, polycation or lipid:nucleic acid conjugates, naked DNA, artificial virions, and agent-enhanced uptake of DNA. Sonoporation using, e.g., the Sonitron 2000 system (Rich-Mar) can also be used for delivery of nucleic acids.

Additional exemplary nucleic acid delivery systems include those provided by Amaxa Biosystems (Cologne, Germany), Maxcyte, Inc. (Rockville, Md.), BTX Molecular Delivery Systems (Holliston, Mass.) and Copernicus Therapeutics Inc, (see for example U.S. Pat. No. 6,008,336). Lipofection is described in e.g., U.S. Pat. Nos. 5,049,386; 4,946,787; and 4,897,355) and lipofection reagents are sold commercially (e.g., Transfectam.TM. and Lipofectin.TM.). Cationic and neutral lipids that are suitable for efficient receptor-recognition lipofection of polynucleotides include those of Feigner, WO 91/17424, WO 91/16024.

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids encoding engineered ZFPs, TALEs and/or CRISPR/Cas systems take advantage of highly evolved processes for targeting a virus to specific cells in the body and trafficking the viral payload to the nucleus. Viral vectors can be administered directly to patients (in vivo) or they can be used to treat cells in vitro and the modified cells are administered to patients (ex vivo).

Vectors (e.g., retroviruses, adenoviruses, liposomes, etc.) containing nucleases and/or donor constructs can also be administered directly to an organism for transduction of cells in vivo. Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

It will be apparent that the nuclease-encoding sequences and donor constructs can be delivered using the same or different systems. For example, the nucleases and donors can be carried by the same DNA MC. Alternatively, a donor polynucleotide can be carried by a MC, while the one or more nucleases can be carried by a standard plasmid or AAV vector. Furthermore, the different vectors can be administered by the same or different routes (intramuscular injection, tail vein injection, other intravenous injection, intraperitoneal administration and/or intramuscular injection. The vectors can be delivered simultaneously or in any sequential order.

Effects of gene manipulation using the methods disclosed herein can be observed by, for example, Northern blots of the RNA (e.g., mRNA) isolated from the tissues of interest. Typically, if the mRNA is present or the amount of mRNA has increased, it can be assumed that the corresponding transgene is being expressed. Other methods of measuring gene and/or encoded polypeptide activity can be used. Different types of enzymatic assays can be used, depending on the substrate used and the method of detecting the increase or decrease of a reaction product or by-product. In addition, the levels of polypeptide expressed can be measured immunochemically, i.e., ELISA, RIA, EIA and other antibody based assays well known to those of skill in the art, such as by electrophoretic detection assays (either with staining or western blotting).

### V. Temperature Shift

The present invention involves subjecting the host cells to a period of cold shock after introduction of the nuclease(s) and/or donor nucleic acids. The cells may be shifted from 37 °C to a lower temperature (cold-shock) within minutes after transfection or may be maintained at 37 °C for a short period of time (1 day for example) prior to shifting to the lower temperature.

The period of time for which the cells are cold shocked can range from hours to days. In certain embodiments, the cells are cold-shocked for between 1 and 4 days. It will be apparent that the period of cold shock will also vary depending on the cell type into which the nuclease is introduced.

Likewise, the temperature at which the cells are cold-shocked is any temperature that reduces cell division, but at which the nuclease(s) is (are) expressed and/or active. Suitable temperatures will vary depending on the host cell type. For mammalian cells, cold shock temperatures include, but are not limited to, 35 °C, 34 °C, 33 °C, 32 °C, 31°C, 30 °C, 29 °C, 28 °C, 27 °C, 26 °C, 25 °C, and even lower. According to the invention, the cells are subjected to a temperature shift from 37 °C to a lower temperature which is between 28 °C and 35 °C. Furthermore, the temperature can vary during the period of cold-shocking, so long as it remains low enough so that the cells are not dividing or are dividing at a reduced rate.

The present invention is further illustrated by the following examples.

### EXAMPLES

### Example 1

### Cold Shock Increases The Frequency Of Homologous Directed Repair For Gene Editing Induced Pluripotent Stem Cells

### Introduction

One of the most promising applications of the Clustered Regularly Spaced Palindromic Repeats (CRISPR) technology is its use in creating genetic models of human disease. CRISPR technology can be used on induced pluripotent stem cells (iPSC) isolated from normal individuals to study a disease phenotype, or on IPSCs derived from disease patients to revert putative disease-causing mutations back to wild type (1, 2). The relative robustness of the CRISPR approach compared to zinc finger nucleases (ZFNs) and transcriptional activator-like effector nucleases (TALENs) has made testing accessible on protein coding mutations as well as empirical data generated by genome-wide association studies and other non-coding mutations (3, 4). Despite numerous successes, gene editing in iPSCs is challenged by the fact that homology directed repair (HDR), the process by which exogenous donor DNA is used to repair CRISPR-induced double strand breaks, is less efficient in iPSCs than transformed cancer cell lines (5-8).

To overcome low HDR rates, researchers have adopted several strategies such as including antibiotic resistance genes on the CRISPR plasmid and/or donor DNA (9) which, while effective, leaves an undesired insertion of foreign DNA into the genome. Combining positive selection markers with technologies that allow for excision of the selectable marker, such as the Cre/lox system or the footprint-free PiggyBAC transposon, represent notable improvements but extend timelines as clonal selection becomes a two-step process (2, 10). Methods that employ single-stranded oligo DNA nucleotide (ssODN) donor molecules avoid the issues that larger, double-stranded DNA molecules present with respect to random integration and unwanted 'footprints', but again are subject to the relatively low frequency of successful repair and sequence conversion around the site of the double-stranded break (7, 11). To contend with the difficulty of isolating rare clones, Miyaoka and colleagues devised a strategy using droplet digital PCR, pools of clones, and sib selection to enrich for extremely rare clones (12). Additional strategies to increase the rate of HDR include timing the delivery of the Cas9 RNP complex to the nuclease by inducing cell cycle synchronization with known chemical inhibitors of cell cycle progression (13). Here, while notable increases in HDR, up to 38%, can be achieved with synchronized HEK293 cells, synchronization had minimal effect in human primary fibroblasts or H9 human embryonic stem cells. Specifics of the ssODN structure and composition have also been shown to affect HDR rates. Lin et al. found that oligos with homology arms of at least 60 nucleotides were most effective, but that strand complementarity was not a factor. A more detailed investigation into how the structure of donor oligos affects HDR in HEK293 cells by Richardson et al. used insights gained from in vitro binding studies of Cas9 RNP-dsDNA complex. Using a GFP-reporter assay, they demonstrated that asymmetric donor oligos that are shorter with respect to the PAM site and are complementary to the (+) strand (i.e., non-target strand) were more effective in promoting HDR than symmetric donor oligos (14). A study optimizing HDR in iPSCs by Paquet et al. showed that efficient insertion of an intended mutation could be achieved with oligos that where complementary to the target strand (-) and that the frequency of the intended mutation integration was distance-dependent from the CRISPR cut site. Fidelity of HDR could also be increased by the introduction of silent base changes into the oligo that disrupted the CRISPR recognition sequence (15).

We have carried out a systematic evaluation of the gene-editing steps in iPSCs to determine the best combination of delivery, CRISPR modality, and donor oligo design. We then tested the effects of a moderate `cold shock' on the cells' ability to carry out HDR. Our optimized method can successfully introduce desired genetic alterations into the genome with 10-30% efficiency through our novel combination of lipids designed for large RNA molecule delivery in conjunction with Cas9-encoding mRNA, symmetric donor oligos complementary to the target strand (-), and silent changes to prevent re-editing. Additional exposure of cells to a brief `cold shock' of 32 °C can increase the amount of perfect HDR as much as two- to ten-fold in instances where low efficiency repair is observed at 37 °C.

### Methods and Materials

### 1) Cell lines and cell culture.

Human mc-iPS Cells were from System Biosciences (SC301A-1) and were maintained on Matrigel (BD Bioscience) coated plates in mTeSR media (Stem Cell Technologies) and 50 units/ml penicillin-streptomycin (Thermo Fisher Scientific) with daily medium change) (Ludwig, T. E., et al. (2006). "Feeder-independent culture of human embryonic stem cells." Nat Methods 3(8): 637-646). For passaging, the cells were washed with PBS and treated with Accutase (Thermo Fisher Scientific) at 37 °C for 5 min. The cells were re-suspended in mTeSR media, centrifuged at 80g for 5 min and cell pellets were re-plated in mTeSR media supplemented with 10 µM ROCK Inhibitor Y-27632 (Cayman Chemical).

### 2) CRISPR and Cas9 Reagents.

CRISPR guides RNAs were designed using the Doench's algorithm (http://portals.broadinstitute.org/gpp/public/) and Zhang laboratory CRISPR design tool (http://crispr.mit.edu). The guide sequences were either subcloned into plasmid pX458 (GenScript) or synthesized as IVT sgRNAs (Thermo Fisher Scientific). GeneArt^{™} Platinum^{™} Cas9 Nuclease was obtained from Thermo Fisher Scientific and Cas9 mRNA (5meC, Ψ) was obtained from TriLink BioTechnologies. The repair templates (Ultramer, IDT) were designed as single-stranded oligonucleotide (ssODN) with target mutations to the middle of the oligonucleotide with homologous genomic flanking sequence on the both side of mutations (Miyaoka, Chan et al. 2014, Richardson, Ray et al. 2016). In some ssODN designs, silent mutations were also introduced at guide RNA binding sequence and PAM site. PCR primers were designed using PRIMER 3 and primers were purchased from Sigma. For sequences of the primers, probes and oligonucleotide donors, see Table 1.

**Table 1: gRNA and oligonucleotides used in this study**

| **Name** | **Target** | **Forward (5' to 3')** | **Reverse (5' to 3')** |
|---|---|---|---|
| CAMK2D-F and CAMK2D-R | CAMK2D | TGGGTTTCCAGGAAGAATTG | TCCCTCTCAAAAGCAAAAGG |
| TGFBR1-F and TGFBR1-R | TGFBR1 | GGTTTACCATTGCTTGTTCAGAG | TGCCCTAAACTAAACCAACAAA |

| **Primers used for droplet digital PCR.** | | | |
|---|---|---|---|
| **Name** | **Target** | **Forward (5' to 3')** | **Reverse (5' to 3')** |
| CAMK2D-ddPCR primer F & R | CAMK2D | GCATTCTCAGTGGTGAGAAGATGT | CAGACCAAGAAGCATTCAGGAA |

| **Probes used for droplet digital PCR** | | | |
|---|---|---|---|
| **Name** | **Target** | **Probe for wild type allele** | **Probe for mutant type allele** |
| CAMK2D-ddPCR probe | CAMK2D | ATGCTGCCAAAATTATCAA | AAAAGCTCTCCGCAAGAG |

| **gRNA sequence** | | | |
|---|---|---|---|
| **Name** | **Target** | **Sequence (5' to 3')** | **PAM sequence** |
| **CAMK-CR1** | CAMK2D | ACACCAAAAAGCTTTCTGCT | AGG |
| **CAMK-CR2** | CAMK2D | AAAAGCTTTCTGCTAGGGGT | GGG |
| **TR-CR2** | TGFBR1 | GTTTGGAGAGGAAAGTGGCG | GGG |
| **TR-CR3** | TGFBR1 | AGAACGTTCGTGGTTCCGTG | AGG |

| **Single-stranded oligonucleotide used in HR** | | | |
|---|---|---|---|
| **HR ssODN** | **Target** | **Sequence (5' to 3')** | |
| C-CR2 | CAMK2D | TTGGTTTCCAGGGGGGCATTCTCAGTGGTGAGAAGATGTATGAAAATTCCTACTGGACAAGAATATGCTGCC AGGATTATCAACACCAAAAAGCTCTCCGCAAGAGGTGGGTATTTTCAACCACATATATTGGTTAATTTTGTATT TGTCATGTTGATTATAGGTTCTGTTGTATGT | |
| C-CR2-Asym | | CAMK2D | TTATAACTACATGATATATTTACATACAACAGAACCTATAATCAACATGACAAATACAAAATTAACCAATATAT GTGGTTGAAAATACCCACCTCTTGCGGAGAGCTTTTTGGTGTTGATAATCCTGGCAGCATATTCTTGTCCAGT AGGAATTTTCATAC |
| T-CR2 | | TGFBR1 | CTTTAGGTTTACCATTGCTTGTTCAGAGAACAATTGCGAGAACTATTGTGTTACAAGAAAGCATTGGCAAAGG CCGATTTGGAGAAGTTTGGAGgGGcAAaTGGCGGGGAGAAGAAGTTGCTGTTAAGATATTCTCCTCTAGAGA AGAACGTTCGTGGTTCCGTGAGGCAGAGAT |
| T-CR2-Asym | | TGFBR1 | TAACATTACAGTTTGATAAATCTCTGCCTCACGGAACCACGAACGTTCTTCTCTAGAGGAGAATATCTTAACA GCAACTTCTTCTCCCCGCCATTTGCCCCTCCAAACTTCTCCAAATCGGCCTTTGCCAATGCTTTCTTGTAACACA ATAGTTCTC |
| T-CR3 | | TGFBR1 | GCATTGGCAAAGGTCGATTTGGAGAAGTTTGGAGAGGAAAGTGGCGGGGAGAAGAAGTTGCTGTTAAGAT ATTTTCCTCTAGAGAAGAACGTTCGTGGTTTCGAGAAGCAGAGATTTATCAAACTGTAATGTTACGTCATGAA AACATCCTGGGATTTATAGCAGCAGACAATAAAGGT |
| T-CR3-Asym | | TGFBR1 | AGCAAATGTTACAGACCTTTATTGTCTGCTGCTATAAATCCCAGGATGTTTTCATGACGTAACATTACAGTTTG ATAAATCTCTGCTTCTCGAAACCACGAACGTTCTTCTCTAGAGGAAAATATCTTAACAGCAACTTCTTCTCCCC GCCACTTT |

### 3) Transfection

For lipid-based transfection of Cas9 mRNA and IVT gRNA in mc-iPSC, the procedures were similar to IVT gRNA and Cas9 protein transfection with minor modification (see Supplementary Methods). Specifically, 480 ng of IVT gRNA and 2 µg of Cas9 mRNA were first mixed in 50 µl of OptiMEM medium and followed by adding 2.5 µl of mRNA-In Stem or Edit-Pro^{™} (MTI-GlobalStem). For homology directed repair experiments, various amount of ssODNs were added to the complex before lipid addition. 100 ng of GFP mRNA was also spiked into each mixture to monitor the transfection efficiency. The plate was incubated at 37 °C for 48h in a 5% CO₂ incubator and the cells were then harvested for genomic DNA extraction.

### 4) Genomic DNA extraction and PCR amplification of edited regions

For genomic DNA extraction from transfected cells, the media from each well was aspirated and the cells were treated with 250 µl of Accutase (Thermo Fisher Scientific) at 37 °C for 10 min. 750 µl of mTeSR media was added to each well and the cells suspension were transferred to a 1.5 ml Eppendorf tube and spun at 1000 g for 5 min. The genomic DNA was extracted using DNeasy Blood & Tissue Kit (QIAGEN) and 100 ng of genomic DNA was used for PCR using Q5 polymerse (NEB) and target specific primers (Table 1). Specifically, PCR amplification of CAMK2D locus was done using primer Camk2D-F and Camk2D-R. PCR amplification of TGFBR1 locus was done using primer TGFβR1-F and TGFβR1-R. The thermocycler condition was set for one cycle of 98 °C for 30s, 31 cycles of 98 °C for 10s, 63 °C for 30s, 72 °C for 1 minute and one cycle of 72 °C for 1 min. The PCR reaction was finally held at 4 °C.

### 5) Next generation sequencing and analysis

PCR Amplicons were cleaned for library preparation by removing high molecular weight (HMW) genomic DNA and residual primers in a two-step cleanup. HMW DNA was removed by adding 0.6 v/v ratio Ampure XP beads (Beckman Coulter), transferring cleared supernate to new plate. Primers were removed by adding 0.2 v/v Ampure XP beads to the transferred supernatant, placed on magnet again until clear, then discard the supernatant. Beads were washed 2x with 80% EtOH, air dried, and resuspended in 20µl H₂O to elute the DNA. Products were monitored for size by Tapestation HSD5000 (Agilent Technologies) and quantified with a Qubit HS DNA (Invitrogen). The cleaned PCR product was used for Nextera XT kit (Illumina) modified to follow usage of one-half of the manufacturer's standard reagent volumes. Samples were uniquely indexed with up to 384 unique i5/i7 combinations using Illumina standard indexing kits. Amplification was carried out with heated lid for 72 °C for 3min, 98 °C for 1 min, then 12-14 cycles of 98 °C for 30s, 55 °C for 30s, 72 °C for 1min, followed by 5 min final extension at 72 °C, and cooled to 4 °C. Libraries were size selected according to the same Ampure XP bead protocol as described above, and eluted in 15µl H₂O. Products were run on Tapestation HSD1000 (Agilent) and quantified by qPCR using KAPA Library Quantification kit for ABI (Kapa Biosystems). Libraries were normalized to 4 nM each in TE pH 8.0 following KAPA Library Quantification Data Analysis Template for Illumina (Kapa Biosystems), and pooled by volume in appropriate ratios. Libraries were denatured and diluted to 12 pM following standard Illumina protocol, 1% v/v PhiX control was spiked in. Run parameters were set at 150bp paired-end, dual indexed 8bp each, and MiSeq 300v2 reagent kit (Illumina) was used. Samples were demultiplexed using MiSeq Reporter v2.6 or bcl2fastq v2.17. Target coverage at the guide site following deduplicating of reads was set at ~300x for clonal samples, and 3000x minimum for evaluating diverse non-clonal populations.

NGS Data analysis was performed using an in-house developed pipeline. Briefly, quality filtering was performed on paired-end reads using PRINSEQ. Filtered reads were then aligned to reference genome with BWA, followed by realignment using ABRA (assembly-based realigner) to enhance indel detection. For quality assurance, we examined the coverage depth in amplicon, and surveyed the whole amplicon region for insertion and deletion frequencies. To calculate the indel frequency in CRISPR site, we used sgRNA sequence (18-20 bases) as target window to count the numbers of wild-type and indel reads spanning this window. In addition, an indel read must have at least one inserted or deleted base inside this window, whereas a wild-type read has no indel in the window, regardless of point mutations. Besides the total percentage of indels, the percentage of in frame indel was calculated to assess indel's disruptiveness (Mose, L. E., et al. (2014). "ABRA: improved coding indel detection via assembly-based realignment." Bioinformatics 30(19): 2813-2815). Indel length histogram as well as all other charts were plotted using R. We also examined point mutation frequencies in sgRNA guide region and its flanking regions. For homology-directed repair (HDR) projects, we categorized oligo types to assess HDR efficiency.

### 6) ddPCR assay to detect CAMK2D wild type and mutation sequence

The QX200TM Droplet Digital PCR System (Bio-Rad laboratories, CA) was used as instructed by the manufacturer. ddPCR assays for detecting CAMK2D wild type and mutation sequence were designed using Primer Express and ordered from Life Technologies (Life Technologies, CA, USA). ddPCR reactions were assembled using standard protocols as follows. ddPCR Super mix for Probes (no dUTP) (Bio-Rad laboratories, CA, USA) was combine with 160 ng of sample genomic DNA, 1 µl of 20× FAM assay and 1 µl of 20× VIC assay (1× CAMK2D-ddPCR primer F & CAMK2D-ddPCR primer R at 900 nM each, 1× probes at 250 nM each), 5 units of restriction enzyme BamHI -HF^{®} (New England BioLabs, MA), and water for a final reaction volume of 20 µl. Reactions were converted into approximately 20,000 one-nanoliter droplets using the QX200 Droplet Generator and transferred to a 96-well plate for thermal cycling per manufacturer recommendation for this Supermix. After thermal cycling, droplets were read on the QX200 Droplet Reader and assigned as positive or negative based on fluorescence amplitude. The primer and probe sequence are listed in Table 1.

### 7) "Cold shock" experiment on transfected cells

One day's prior to transfection, the mc-IPSCs were seeded in 24 well plate as described in transfection section and divided into four groups (P1-P4), group P1 to group P3 were kept at 37 °C and group P4 was incubated 32 °C for 24 hours. The cells were then transfected with IVT gRNA/Cas9 mRNA and ssODN using 'Edit-Pro' as described. After transfection, group P1 was kept at 37 °C until harvested while the rest of groups were transferred to 32 °C until harvested with the exception of group P3 which was moved back to 37 °C 24 hours post transfection. The cells were harvested for genomic DNA isolation after 48 hours as described and Indel formation or HDR were measured by either ddPCR or NGS.

### 8) Additional Transfection Methods

For lipid-based transfection of DNA in mc-iPSCs, the cells were seeded in Madrigal coated 24-well plate at 1×10⁵ per well one day prior to the transfection. On the day of transfection, 1 µg of pX458-CRISPR DNA was diluted in 50 µl of OptiMEM medium and followed by adding of 2 µl of DNA-In^{®} Stem transfection reagent (MTI-GlobalStem). For homology directed repair experiments, various amount of ssODNs were added to the mixture before lipid addition. The samples were gently mixed and incubated at room temperature for 15min. The entire mixture was then added to the cells drop by drop. The plate was incubated at 37 °C for 48hs in a 5% CO2 incubator and the cells were then harvested for genomic DNA extraction.

For lipid-based transfection of IVT gRNA and Cas9 Nuclease in mc-iPSCs, the procedures were similar to DNA transfection with minor modification (Liang, X., et al. (2015). "Rapid and highly efficient mammalian cell engineering via Cas9 protein transfection." J Biotechnol 208: 44-53). Specifically, 480 ng of IVT gRNA and 2 µg of Cas9 Nuclease were first mixed in 50 µl of OptiMEM medium and keep at room temperature for 10min to form a stable RNP complex followed by addition of 2.5 µl of mRNA-In Stem or Edit-Pro (MTI-GlobalStem). For homology directed repair experiments, various amount of ssODNs were added to the complex before lipid addition. 100 ng of GFP mRNA was also spiked into each mixture to monitor the transfection efficiency. The plate was incubated at 37 °C for 48hs in a 5% CO2 incubator and the cells were then harvested for genomic DNA extraction.

For Nucleofection of pX458 CRISPR plasmid with or without ssODN, mc-iPSC were first cultured in Matrigel-coated 10 mm dish until reach 60-70% confluent. The cells were washed with PBS and treated with 3 ml of Accutase (Thermo Fisher Scientific) at 37 °C for 5-8 min until all cells were dissociated. The cells were re-suspended in mTeSR media and counted. The cells were then transferred to 15 ml tube and spun down at 80g for 5 min. After the supernatant was removed, the cells were re-suspended in P3 or P4 Nucleofection solution (Lonza, Basel Switzerland) at 1×10⁷ per ml. 20 µl of the cell suspension were transferred to a tube and 1 µg of pX458-CRISPRs were added to each tube. For homology directed repair experiment, various amount of ssODNs were also added to the mixture. The suspension were then transferred to each well of 8 well strip (Lonza, Basel Switzerland) with care to avoid generate bubbles and electroporated using Amaxa^{™} 4D-Nucleofector^{™} (Lonza, Basel Switzerland) with program CM-113 or CE-118. The Nucleofected cells were directly plated into individual well of Matrigel-coated 24-well plate which contained 500 µl of pre-warmed mTeSR media with 10 µM of ROCK Inhibitor Y-27632 in each well. The plate was incubated at 37 °C for 48h in a 5% CO2 incubator and the cells were then harvested for Genomic DNA extraction.

For Nucleofection of IVT gRNA and Cas9 protein in mc-iPSCs, the procedure was similar to DNA Nucleofection with minor modification. Specifically, 480 ng of IVT gRNA and 2 µg of Cas9 protein were first mixed together in OptiMEM medium to final volume of 5 µl and keep at room temperature for 10 min to form stable RNP complex. For homology directed repair experiments, various amount of ssODNs were also added to the complex. The complex was then transferred to 20 µl of cells suspension in P3 or P4 nucleofection solution and electroporated using Amaxa^{™} 4D-Nucleofector^{™} (Lonza, Basel Switzerland) with program CM-113 or CE-118 as described above.

### Results

### I. Efficient HDR in iPSCs using CRISPR/Cas9 RNA modality and lipid delivery.

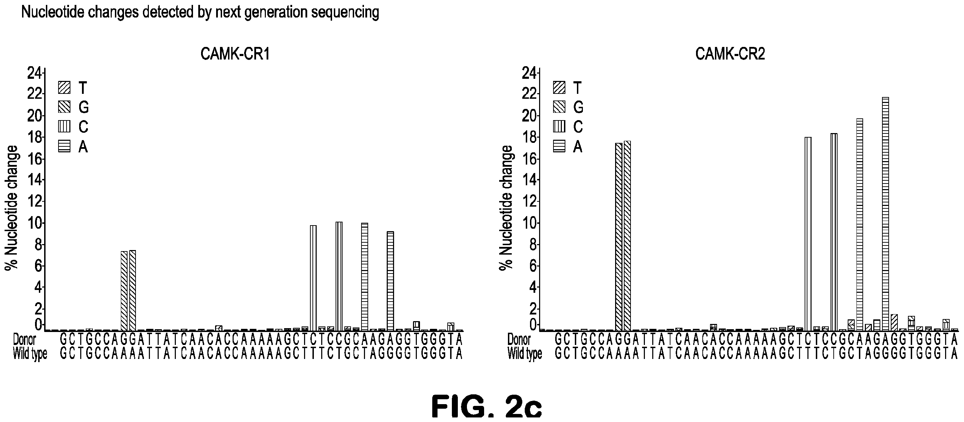

We evaluated several aspects of gene editing protocols in order to find the best conditions for generating HDR within the CAMK2D gene. First, we determined which CRISPR modalities (e.g., all-in-one plasmid DNA, sgRNA and Cas9 mRNA, or sgRNA in vitro transcription (IVT)/Cas9 ribronucleoprotein) and delivery methods (e.g., nucleofection or lipids formulated for enhanced delivery of large DNA and RNA molecules) generated the greatest the number of double-stranded breaks at two specific locations within the CAMK2D gene (Figure 1a) as detected by PCR amplicon next-generation sequencing (NGS). The best NHEJ-induced indel rate for each modality and delivery method are presented in Table 2. The complete matrix of conditions used to determine optimal indel formation were then re-tested to determine the best combination of modality and delivery to promote HDR are also in Table 2. We used a multiplexed ddPCR assay to measure the incorporation of four base changes designed to disrupt the CRISPR recognition sequence and, by proxy, the incorporation of the specific mutations designed to create a kinase-dead version of CAMK2D on the same oligo (Figure 1b). The amount of wild type unedited sequence was determined using a different probe that specifically detected the non-HDR wild type alleles (Figure 1b). The donor oligo design was symmetric with respect to the lengths of the homology arms and the CRISPR cleavage sites were located as close as possible to the intended kinase dead mutations. The donor sequence was also homologous to the non-targeting CRISPR cut strand (+). The four silent mutations introduced by the oligo altered the CRISPR recognition sequence for guide CAMK-CR2 in four positions and in guide CAMK-CR1, mutated the PAM sequence and introduced 3 sequence changes. The assay was validated using both synthesized fragments of the different DNA sequences, and on clones previously made in HEK293 cells known to be heterozygous and homozygous for the HDR donor oligo sequence (data not shown). The best HDR rate for all comparisons are present in Table 2, and the data for the best combination presented in Figure 2a. For, IVT sgRNA/Cas9 mRNA and EditProTM lipid, we observed 9% of all alleles incorporating the donor oligo sequence for CAMK-CR1, and 19% of the alleles for CAMK-CR2 (Figure 2b). To confirm ddPCR results, we performed NGS on PCR amplicons from the transfected populations of iPSCs (Fig. 2c). For IVT sgRNA/Cas9 m RNA with both CAMK-CR1 and CAMK-CR2, the intended base changes that would indicate successful HDR into the locus were observed at their precise genomic coordinates and at frequencies that closely matched those determined by ddPCR, including the two guanine substitutions that were not directly measured by the ddPCR assay. These two substitutions, which are more distal to the CRISPR cut sites than the silent changes designed to disrupt sgRNA annealing, were observed at lower frequencies.

**Table 2 Best Indel and HDR Rate of different delivery and CRISPR modalities conditions**

| **Transfection methods** | **Nucleofection** | | | **Lipid** | | | | | |
|---|---|---|---|---|---|---|---|---|---|
| **sgRNA format** | **DNA format** | **RNA format** | | **DNA format** | | **RNA format** | | | |
| **Cas9/sgRNA source** | **All-in-one** | **sgRNA/Cas9 RNP** | **sgRNA/Cas9 mRNA** | **All-in-one** | | **sgRNA/Cas9 RNP** | | **sgRNA/Cas9 mRNA** | |
| **Transfection reagents or instrument** | **4D-Nucleofector^{™}** | | | **DNA-In^{®} Stem** | **DNA-In^{®} CRISPR** | **mRNA-In^{®} Stem** | **EditPro^{™}** | **mRNA-In^{®} Stem** | **EditPro^{™}** |
| **Best Indel rate** | 29.5%^{b,f} | 7.7%^{a} | 1.0%^{a} | 43.5%^{a} | 28.3%^{a} | 36.3%^{a} | 22.4%^{a} | 26.0%^{a} | 38.8%^{a} |
| **Best HDR rate** | 2.6%^{c,d} | 1.0%^{c,d} | *** | 2.9%^{c,d} | 2.7^{c,d} | 3.2%^{e} | 4.5%^{e} | 9.3%^{e} | 26.1%^{e} |

| | | | | | | | | | |
|---|---|---|---|---|---|---|---|---|---|
| *** Not tested due to very low Indel rate a. sgRNA CAMK-CR1 b. sgRNA CAMK-CR2 c. sgRNA CAMK-CR1 and ssODN C-CR2 d. Only sgRNA CAMK-CR1 was tested in these experiments e. sgRNA CAMK-CR2 and ssODN C-CR2 f. sgRNA CAMK-CR1 showed 25.8% Indel rate | | | | | | | | | |

### II. 'Cold shock' increases the rate of HDR.

Based on previous observations that a T-antigen temperature-sensitive immortalized cell line grown and maintained at 32 °C underwent HDR more efficiently than a similar cell line grown at 37 °C (data not shown), we tested if exposing the mc-iPS cell line to various 32 °C intervals would have an effect on the efficiency of HDR. The design of the experiment and the resulting percentages of the total alleles having undergone HDR as measured by ddPCR at each temperature are presented in Table 3. Using normal culturing conditions at 37 °C as the baseline for HDR (Group PL1), HDR frequencies of 7.50% and 5.0% for guide CAMK-CR1, and 16.16% and 8.86% for guide CAMK-CR2 at the 10 pmole and 30 pmole concentrations were observed respectively. When the cells were transferred to 32 °C immediately after transfection and kept at that condition for 24 hours, then moved to 37 °C until for an additional 24 hours (Group PL2) we observed a statistically significant increase of between 1.8 to 2.3 fold in HDR. The effect was more pronounced at the 30 pmole concentration where lower HDR efficiencies were observed at baseline. Exposing the cells to 32 °C for 48 hour post transfection (Group PL3) also had a statistically significant effect on HDR, increasing it from 2.0 to 3.6 fold, with again the effect being more pronounced in conditions where HDR was lower at baseline.

**Table 3. HDR efficiencies of CRISPR/ssODN at various temperature at CAMK2D locus**

| **Group** | | **PL1** | | **PL2** | | **PL3** | |
|---|---|---|---|---|---|---|---|
| **Conditions** | | **37°C-37°C-37°C** | | **37°C-32°C-37°C** | | **37°C-32°C-32°C** | |
| **sgRNA** | | **CAMK-CR1** | **CAMK-CR2** | **CAMK-CR1** | **CAMK-CR2** | **CAMK-CR1** | **CAMK-CR2** |
| **ssOND input** | **None** | 0.02±0 | 0.01±0 | 0.02±0 | 0.19±0.07 | 0.00±0.00 | 0.02±0.00 |
| | **10 pmol** | 7.50±0.54^{a} | 16.16±0.76^{c} | 15.85±0.51^{a} | 29.40±0.81^{c} | 19.46±0.47^{a} | 33.24±1.53^{c} |
| | **30 pmol** | 5.02±0.37^{b} | 8.86±1.27^{d} | 13.79±0.40^{b} | 20.70±1.44^{d} | 18.14±0.49^{b} | 27.16±1.63^{d} |

| | | | | | | | |
|---|---|---|---|---|---|---|---|
| The experiments were carried out at different temperatures as described in "material and methods" and HDR efficiencies were determined by ddPCR using probes specific for wild type and mutant sequences at the CAMK2D locus. Data presented as mean percentage of droplets containing the mutant allele± standard error from three independent experiments and eight replicates. The significance of HDR efficiency difference among the three temperature conditions for each gRNA and ssODN treatment were analyzed by one way ANOVA ( a,b,c,d: one way ANOVA P<0.0001, follow-up Dunnett's multiple comparison, PL2 versus PL1: P = 0.0001, PL3 versus PL1: P = 0.0001) | | | | | | | |

### III. 'Cold shock' and alternative single-stranded oligo nucleotide donor designs affect the efficiency of HDR.

Recent data suggest that precise donor oligo design can have dramatic effects on the efficiency of donor oligo HDR. More specifically, oligos that are asymmetric in length with respect to the CRISPR cut site (shorter on the side proximal to the cut site) and with sequence complementarity to the non-targeted strand (the strand not initially cleaved by Cas9) are more efficient promoters of HDR than the design we employed for editing the CAMK2D locus i.e., symmetric around the CRISPR cut site and complementary to the targeted strand (14). To compare the two designs directly and further test the effects of cold shock on HDR, we designed a gene editing experiment whereby we compared the amount of HDR observed with the symmetrical targeted strand oligo donor to that observed with an asymmetrical non-targeted strand oligo donor designed to introduce the same sequence alternations (Fig. 1b). We determined the amount of HDR by amplicon based NGS and analyzed the resulting sequence data in several ways: 1) the amount of total HDR at the locus, i.e., oligo directed repair regardless of whether all or part of the intended changes are present; 2) the percentage HDR that represented `perfect HDR', oligo directed repair with all six intended base changes intact; 3) the percentage of HDR for which the sequence that had putatively undergone re-editing once repaired by virtue of indels being re-introduced into the converted sequence; and 4) the percentage of HDR where partial oligo-directed repair occurred such that sequence is missing the two more distal sequence changes (the intended CAMK2 kinase dead mutations (Figure 3a and Table 4).

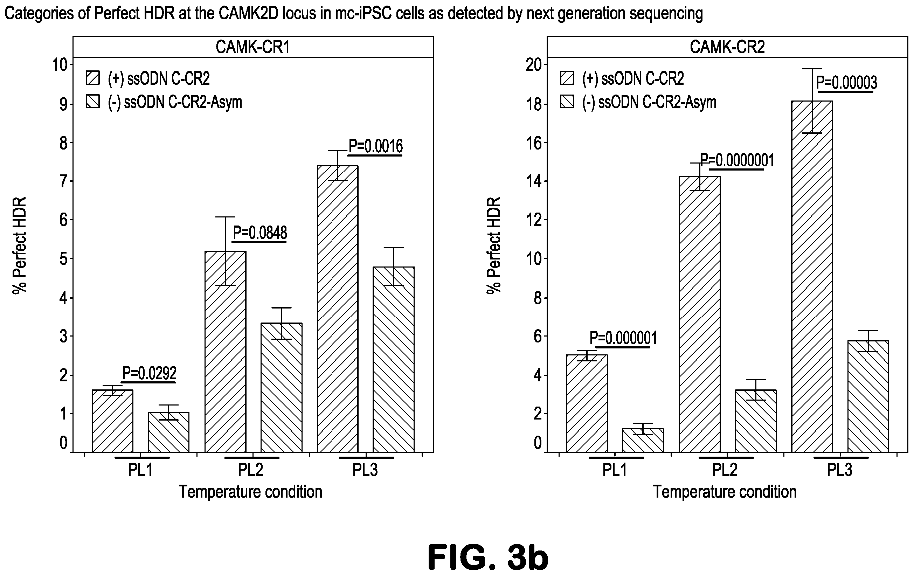

As initially observed by ddPCR, guide CAMK-CR2 was more efficient in promoting total HDR then guide CAMK-CR1 at baseline conditions, i.e., cell transfected and maintained at 37 °C, albeit at lower overall HDR (Figure 2b and Figure 3a). The amount of total HDR across all temperature conditions and oligo concentrations were also comparable for guide CAMK-CR1. In general, statistically significant increases in total HDR were observed across all comparisons of temperature, guide and oligo design (Figure 3a and Table 4). For guide CAMK-CR1 the amount of total HDR for both oligos, across the three temperature conditions was essentially the same and where an approximately 2.9 fold increase in total HDR was observed under the PL3 temperature condition for both oligo designs (Figure 3a). For CAMK-CR2, the fold increase in total HDR for the symmetrical oligo, C-CR2 was approximately 2.4 fold but the magnitude of the response was 40% of the alleles having undergone some type of HDR (Figure 3a). For the asymmetric design, C-CR2, a total increase of HDR of 3.5 fold was observed between PL1 and PL3 however the magnitude of the response was approximately half of that observed with the symmetrical guide (Figure 3a). However, when only considering the amount of 'perfect' HDR, observed as a result of `cold shock', the type of oligo used had a dramatic effect especially for guide CAMK-CR2 where the differential in total HDR was higher between the two designs to begin with and where symmetrical oligo design was superior in directing conversion of all six nucleotide changes (Figure 3b and Table 4). For guide CAMK-CR1, where the amount of total HDR was similar for both oligo types across all temperature conditions, the amount of 'perfect' HDR was again greater for the symmetrical oligo however the difference was only statistically significant under the PL3 conditions (Figure 3b and Table 4).

**Table 4. Effects of `cold shock' and ssODN HDR donor design on HDR efficiency at the CAMK2D locus in mc-iPSCs as determined by NGS**

| | **sgRNA** | **CAMK-CR1** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.11 | 0.02 | 0.04 | 0.01 | 99.85 | 0.02 |
| **PL2** | **37°C-32°C-37°C** | 0 | 0 | 0.15 | 0.05 | 0.06 | 0.01 | 99.79 | 0.06 |
| **PL3** | **37°C-32°C-32°C** | 0.02 | 0.01 | 0.1 | 0.02 | 0.08 | 0.04 | 99.8 | 0.05 |
| | | | | | | | | | |

| | **sgRNA** | **CAMK-CR1** | | | | | | | |
|---|---|---|---|---|---|---|---|---|---|
| | **ssODN** | **C-CR2** | | | | | | | |
| | **ssODN Input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 2.47 | 0.58 | 0.82 | 0.13 | 4.48 | 0.53 | 92.23 | 1.2 |
| **PL2** | **37°C-32°C-37°C** | 6.32 | 0.98 | 1.65 | 0.18 | 9.75 | 0.65 | 82.29 | 1.75 |
| **PL3** | **37°C-32°C-32°C** | 9.09 | 1.12 | 1.96 | 0.16 | 11.15 | 0.51 | 77.8 | 1.29 |
| | | | | | | | | | |

| | **sgRNA** | **CAMK-CR1** | | | | | | | |
|---|---|---|---|---|---|---|---|---|---|
| | **ssODN** | **C-CR2** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 1.75 | 0.48 | 0.8 | 0.21 | 2.96 | 0.44 | 94.5 | 1.05 |
| **PL2** | **37°C-32°C-37°C** | 5.71 | 0.96 | 1.45 | 0.2 | 7.13 | 0.43 | 85.71 | 1.55 |
| **PL3** | **37°C-32°C-32°C** | 8.41 | 1.16 | 1.8 | 0.25 | 9.2 | 0.48 | 80.59 | 1.57 |
| | | | | | | | | | |

| | **sgRNA** | **CAMK-CR1** | | | | | | | |
|---|---|---|---|---|---|---|---|---|---|
| | **ssODN** | **C-CR2-Asym** | | | | | | | |
| | **ssODN Input** | **10 pmol** | | | | | | | |
| **Group** | Temperature | (PctPerfectOligo) | | | | | | | |
| | | Average | StdErr | Average | (PctEditedOligo) \| StdErr | Average \| | (PctPartialOligo) StdErr | Average \| | (PctNonOligo) StdErr |
| **PL1** | **37°C-37°C-37°C** | 1.03 | 0.19 | 0.51 | 0.08 | 5.87 | 0.74 | 92.6 | 0.97 |
| **PL2** | **37°C-32°C-37°C** | 3.33 | 0.42 | 0.99 | 0.07 | 13.24 | 0.84 | 82.44 | 1.3 |
| **PL3** | **37°C-32°C-32°C** | 4.79 | 0.48 | 1.16 | 0.14 | 16.59 | 1.09 | 77.46 | 1.62 |
| | | | | | | | | | |

| | **sgRNA** | **CAMK-CR1** | | | | | | | |
|---|---|---|---|---|---|---|---|---|---|
| | **ssODN** | **C-CR2-Asym** | | | | | | | |
| | **ssODN Input** | **30 pmol** | | | | | | | |
| **Group** | Temperature | (PctPerfectOligo) | | (PctEditedOligo) | | (PctPartialOligo) | | | |
| | | Average | StdErr | Average | StdErr | Average | StdErr | Average | (PctNonOligo) StdErr |
| **PL1** | **37°C-37°C-37°C** | 0.89 | 0.16 | 0.46 | 0.06 | 5.28 | 0.52 | 93.37 | 0.71 |
| **PL2** | **37°C-32°C-37°C** | 3.33 | 0.37 | 0.9 | 0.04 | 13.22 | 0.9 | 82.56 | 1.28 |
| **PL3** | **37°C-32°C-32°C** | 5.53 | 0.29 | 1.37 | 0.15 | 17.08 | 1.08 | 76.01 | 1.39 |
| | | | | | | | | | |

| | **sgRNA** | **CAMK-CR2** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.2 | 0.05 | 0.28 | 0.07 | 99.52 | 0.07 |
| **PL2** | **37°C-32°C-37°C** | 0.02 | 0.01 | 0.47 | 0.12 | 0.28 | 0.06 | 99.23 | 0.17 |
| **PL3** | **37°C-32°C-32°C** | 0 | 0 | 0.52 | 0.05 | 0.33 | 0.08 | 99.15 | 0.1 |
| | | | | | | | | | |

| | **sgRNA** | **CAMK-CR2** | | | | | | | |
|---|---|---|---|---|---|---|---|---|---|
| | **ssODN** | **C-CR2** | | | | | | | |
| | **ssODN Input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 6.54 | 1.02 | 1.35 | 0.39 | 9.8 | 0.79 | 82.32 | 2.05 |
| **PL2** | **37°C-32°C-37°C** | 15.73 | 1.1 | 1.95 | 0.43 | 14.27 | 1.27 | 68.05 | 2.18 |
| **PL3** | **37°C-32°C-32°C** | 20.05 | 1.74 | 2.16 | 0.45 | 17.96 | 1.83 | 59.83 | 3.24 |
| | | | | | | | | | |

| | **sgRNA** | **CAMK-CR2** | | | | | | | |
|---|---|---|---|---|---|---|---|---|---|
| | **ssODN** | **C-CR2** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 4.2 | 1.36 | 1.01 | 0.41 | 4.34 | 0.9 | 90.46 | 2.63 |
| **PL2** | **37°C-32°C-37°C** | 10.12 | 1.83 | 1.4 | 0.43 | 9.25 | 1.08 | 79.23 | 3.31 |
| **PL3** | **37°C-32°C-32°C** | 14.77 | 2.23 | 1.79 | 0.4 | 12.32 | 0.54 | 71.12 | 2.85 |
| | | | | | | | | | |

| | **ssODN** | **C-CR2-Asym** | | | | | | | |
|---|---|---|---|---|---|---|---|---|---|
| | **ssODN Input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 1.24 | 0.29 | 0.2 | 0.05 | 5.16 | 1 | 93.4 | 1.32 |
| **PL2** | **37°C-32°C-37°C** | 3.21 | 0.54 | 0.46 | 0.1 | 9.28 | 1.15 | 87.06 | 1.76 |
| **PL3** | **37°C-32°C-32°C** | 5.72 | 0.54 | 0.52 | 0.08 | 14.93 | 0.49 | 78.83 | 0.65 |
| | | | | | | | | | |

| | **sgRNA** | **CAMK-CR2** | | | | | | | |
|---|---|---|---|---|---|---|---|---|---|
| | **ssODN** | **C-CR2-Asym** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 1.37 | 0.21 | 0.23 | 0.06 | 4.94 | 0.43 | 93.46 | 0.67 |
| **PL2** | **37°C-32°C-37°C** | 5.61 | 0.71 | 0.79 | 0.35 | 15.75 | 1.65 | 77.85 | 1.14 |
| **PL3** | **37°C-32°C-32°C** | 9.84 | 0.49 | 0.85 | 0.09 | 23.7 | 2.13 | 65.61 | 2.39 |

To extend these observations to another locus and to further test the effects of `cold shock' and oligo design on HDR, we designed a gene editing experiment to insert silent changes and SNPs into the TGFRB1 locus. The locations of the two guides tested are shown in Figure 4a and the sequences of the four donor oligos, the positions of the intended sequence alterations and their relationship to the guide positions are shown in Figures 4b and 4c. Guide TR-CR2, was designed to direct cleavage approximately 31 bps 3' to the intended A to C sequence change (Figure 4b). Both symmetric and asymmetric donor oligos also contained 3 additional sequence changes designed to disrupt guide recognition and re-editing at the locus. The length of the homology arms are also listed in Figure 4b. Guide TR-CR3 was designed to direct cleavage approximately 30 bps 3' from the intended C to T sequence change (Figure 4c). As with TR-CR2 and its ssODNs, three additional silent sequence alterations were also included to prevent re-editing of the converted locus. The length of the homology arms was designed to be as close as possible to the ssODNs used for guide TR-CR2 (Figure 4c).

Using the IVT sgRNA/Cas9 RNA lipid format, both CRISPRs were efficient at generating indels in the mc-iPSC line, where, in the absence of repair oligos, the percent of alleles with indels as determined by NGS were 92% for TR-CR2 and 64% for TR-CR3. In the presence of both repair oligos, guide TR-CR2 led to very efficient total HDR rates of 60% for the symmetric oligo T-CR2 and 42% for the asymmetric design at condition PL1, 37 °C (Figure 5a and Table 5). For guide TR-CR3, total HDR percentages of 41 and 34 were observed at 37 °C for the symmetrical and asymmetrical designs respectively (Figure 5a and Table 5).

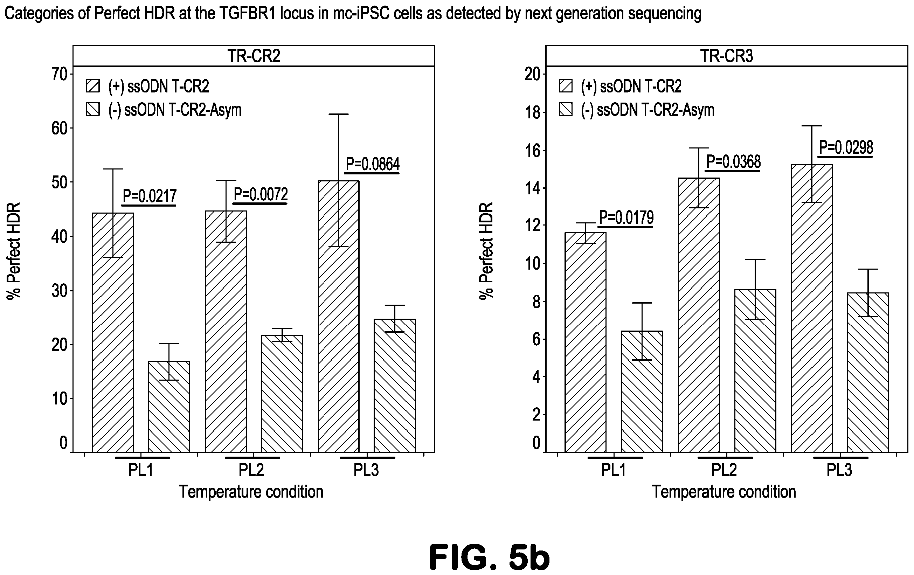

As observed with CAMK2D, culturing the cells at 32 °C for either 24 hours (Group PL2) or 48 hours (Group PL3) produced an increase in HDR; however, the effect was generally minimal given the relativity high rates of HDR to begin with at 37 °C and for the most part did not reach statistical significance (Figure 5a and Table 5). However, the `cold shock' effect was more pronounced for the asymmetric oligos where the amount of `perfect HDR' at 37 °C was less than the symmetric oligos. Here, statistical significance was achieved for guide TR-CR2 and asymmetric donor T-CR2 when the amount of total HDR observed at 37 °C (PL1) was compared to the amount observed when the cells were culture at 32 °C for 48 hours (PL3) and for guide TR-CR3 and the asymmetric guide T-CR3 at both the PL2 and PL3 conditions (Figure 5a and Table 5). However when only considering the amount of 'perfect' HDR, observed as a result of `cold shock' as well as at baseline conditions, the type of oligo used had a dramatic effect (Figure 5b and Table 5). Across all comparison but one (TR-CR2/PL3), the symmetrical donor oligos were statistically significantly superior at directing 'perfect' HDR repair than their asymmetric counterparts (Figure 5b and Table 5).

**Table 5. Effects of 'cold shock' and ssODN HDR donor design on HDR efficiency at the TGFBR1 locus in mc-iPSCs as determined by NGS**

| | **SgRNA** | **TR-CR2** | | | | | | | |
|---|---|---|---|---|---|---|---|---|---|
| | **ssODN** | **None** | | | | | | | |
| | **ssODN input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.41 | 0.2 | 0.14 | 0.07 | 99.45 | 0.25 |
| **PL2** | **37°C-32°C-37°C** | 0 | 0 | 0.72 | 0.36 | 0.08 | 0.03 | 99.19 | 0.39 |
| **PL3** | **37°C-32°C-32°C** | 0.15 | 0.15 | 0.42 | 0.18 | 0.17 | 0.06 | 99.26 | 0.22 |
| | | | | | | | | | |

| | **sgRNA** | **TR-CR2** | | | | | | | |
|---|---|---|---|---|---|---|---|---|---|
| | **ssODN** | **T-CR2** | | | | | | | |
| | **ssODN input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 44.3 | 8.24 | 3.37 | 1.18 | 11.97 | 2.22 | 40.37 | 7.07 |
| **PL2** | **37°C-32°C-37°C** | 44.68 | 5.62 | 2.92 | 0.51 | 11.74 | 1.2 | 40.66 | 4.22 |
| **PL3** | **37°C-32°C-32°C** | 50.31 | 12.2 | 3.65 | 0.64 | 11.91 | 4.14 | 34.14 | 9.13 |
| | | | | | | | | | |

| | **sgRNA** | **TR-CR2** | | | | | | | |
|---|---|---|---|---|---|---|---|---|---|
| | **ssODN** | **T-CR2** | | | | | | | |
| | **ssODN input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 37.96 | 3.31 | 2.72 | 0.59 | 12.25 | 1.14 | 47.07 | 4.63 |
| **PL2** | **37°C-32°C-37°C** | 43.9 | 3.68 | 2.72 | 0.22 | 11.89 | 0.87 | 41.5 | 4.15 |
| **PL3** | **37°C-32°C-32°C** | 53.99 | 10.39 | 2.85 | 1.06 | 7.5 | 2.49 | 35.68 | 7.17 |
| | | | | | | | | | |

| | **sgRNA** | **TR-CR2** | | | | | | | |
|---|---|---|---|---|---|---|---|---|---|
| | **ssODN** | **T-CR2-Asym** | | | | | | | |
| | **ssODN input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 16.83 | 3.42 | 2.05 | 0.48 | 23.38 | 1.53 | 57.74 | 3.89 |
| **PL2** | **37°C-32°C-37°C** | 21.74 | 1.19 | 2.1 | 0.7 | 32.93 | 4.24 | 43.23 | 4.65 |
| **PL3** | **37°C-32°C-32°C** | 24.8 | 2.5 | 2.74 | 0.37 | 28.96 | 2.53 | 43.5 | 2.98 |
| | | | | | | | | | |

| | **sgRNA** | **TR-CR2** | | | | | | | |
|---|---|---|---|---|---|---|---|---|---|
| | **ssODN** | **T-CR2-Asym** | | | | | | | |
| | **ssODN input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 19.71 | 0.88 | 2.04 | 0.34 | 28 | 3.04 | 50.26 | 2.19 |
| **PL2** | **37°C-32°C-37°C** | 27.2 | 1.67 | 2.47 | 0.97 | 26.02 | 1.88 | 44.32 | 2.71 |
| **PL3** | **37°C-32°C-32°C** | 28.23 | 1.9 | 2.53 | 0.71 | 26.02 | 1.62 | 43.23 | 2.89 |
| | | | | | | | | | |

| | **sgRNA** | **TR-CR3** | | | | | | | |
|---|---|---|---|---|---|---|---|---|---|
| | **ssODN** | **None** | | | | | | | |
| | **ssODN input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 2.49 | 0.41 | 1.98 | 0.28 | 95.54 | 0.33 |
| **PL2** | **37°C-32°C-37°C** | 0 | 0 | 3.55 | 0.58 | 0.99 | 0.21 | 95.45 | 0.38 |
| **PL3** | **37°C-32°C-32°C** | 0 | 0 | 3.93 | 0.75 | 1.23 | 0.3 | 94.85 | 0.57 |
| | | | | | | | | | |

| | **sgRNA** | **TR-CR3** | | | | | | | |
|---|---|---|---|---|---|---|---|---|---|
| | **ssODN** | **T-CR3** | | | | | | | |
| | **ssODN input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 11.63 | 0.55 | 3.45 | 0.96 | 27.21 | 2.27 | 57.72 | 3.32 |
| **PL2** | **37°C-32°C-37°C** | 14.58 | 1.55 | 5.03 | 0.47 | 30.1 | 3.07 | 50.29 | 3.58 |
| **PL3** | **37°C-32°C-32°C** | 15.26 | 2.04 | 4.72 | 0.38 | 31.61 | 3.5 | 48.41 | 3.58 |
| | | | | | | | | | |

| | **sgRNA** | **TR-CR3** | | | | | | | |
|---|---|---|---|---|---|---|---|---|---|
| | **ssODN** | **T-CR3** | | | | | | | |
| | **ssODN input** | **30 pmol** | | | | | | | |
| **Group** | **Temperature** | | (PctPerfectOligo) | | (PctEditedOligo) | | (PctPartialOligo) | (PctNonOligo) | |
| | | Average | StdErr | Average | StdErr | Average | StdErr | Average | StdErr |
| **PL1** | **37°C-37°C-37°C** | 7.07 | 1.93 | 3.86 | 0.73 | 26.59 | 6.11 | 62.49 | 6.66 |
| **PL2** | **37°C-32°C-37°C** | 8.93 | 2.44 | 4.32 | 1.02 | 33.43 | 7.69 | 53.32 | 5.39 |
| **PL3** | **37°C-32°C-32°C** | 10.02 | 2.2 | 3.66 | 0.62 | 35.27 | 6.99 | 51.05 | 5.08 |

| | **ssODN** | **T-CR3--Asym** | | | | | | | |
|---|---|---|---|---|---|---|---|---|---|
| | **ssODN input** | **10 p mol** | | | | | | | |
| **Group** | **Temperature** | (PctPerf ectOligo) | | (PctEdit edOligo) | | (PctPart ialOligo) | | (PctNo nOligo) | |
| | | Average | StdErr | Average | StdErr | Average | StdErr | Average | StdErr |
| **PL1** | **37°C-37°C-37°C** | 6.42 | 1.52 | 2.79 | 0.67 | 24.83 | 2.61 | 65.96 | 2.71 |
| **PL2** | **37°C-32°C-37°C** | 8.67 | 1.58 | 3.65 | 0.74 | 27.55 | 2.12 | 60.14 | 2.32 |
| **PL3** | **37°C-32°C-32°C** | 8.46 | 1.25 | 3.34 | 0.27 | 28.35 | 2.11 | 59.85 | 1.75 |
| | | | | | | | | | |

| | **sgRNA** | **TR-CR3** | | | | | | | |
|---|---|---|---|---|---|---|---|---|---|
| | **ssODN** | **T-CR3-Asym** | | | | | | | |
| | **ssODN input** | **30 p mol** | | | | | | | |
| **Group** | **Temperature** | (PctPerf ectOligo) | | (PctEdit edOligo) | | (PctPart ialOligo) | | (PctNo nOligo) | |
| | | Average | StdErr | Average | StdErr | Average | StdErr | Average | StdErr |
| **PL1** | **37°C-37°C-37°C** | 4.9 | 1.54 | 4.15 | 1.14 | 33.81 | 4.98 | 57.14 | 4.63 |
| **PL2** | **37°C-32°C-37°C** | 6.49 | 1.88 | 3.32 | 0.74 | 30.4 | 2.34 | 59.78 | 2.78 |
| **PL3** | **37°C-32°C-32°C** | 5.5 | 0.34 | 3.88 | 1.17 | 33.42 | 6.47 | 57.21 | 6.15 |

### IV. 'Cold shock' is more effective when base HDR rates are lower.

To test whether 'cold shock' was effective in a cell type other than the particular mc-iPSC line under investigation, we repeated the identical CAMK2D gene editing experiment used to derive the data in Table 3 in HEK293 cells and determined the levels HDR using ddPCR (Table 6) and specific HDR categories by NGS (Figure 6 and Table 7). Baseline total HDR levels for both CAMK2D sgRNAs and two donor oligo concentrations were approximately 1% at 37 °C (Table 6, Table 7, and Figure 6). These results were obtained using both plasmid based all-in-one CRISPR modality (data not shown) as well as the IVT sgRNA/Cas9RNA modality used for iPSCs in independent experiments. This is in contrast to that observed in the mc-iPSC line where total HDR levels were over 10% and 20% respectively for CAMK-CR1 and CR2 at the 10 pmole concentration even though the HEK293 cell lines was transfected to the same relative efficiencies (Figure 3a and data not shown). The degree by which the locus and the cell type can determine the levels of gene editing have been described by others (8). However, despite the low HDR rates observed at 37 °C, 'cold shock' under both sets of conditions, 24 hours (PL2) and 48 hours (PL3), produced for the best sgRNA, CAMK-CR2, a statistically significant 6.9-fold increase in total HDR at both the 24 hour and 48 hour conditions as determined by ddPCR (Table 6). Both guides and all conditions produced statistically significant increases in total HDR in response to `cold shock' (Figure 6 and Table 7).

Amplicon based NGS and analysis confirmed that `cold shock' produced statistically significant increase in total HDR for both guides with the exception of the PL1 vs PL3 comparison for guide CAMK-CR2. In general, increases in 'perfect' HDR exceeded 5 to 20 fold for both guides and all conditions (Figure 6 and Table 7).

**Table 6. HDR efficiencies of sgRNA/ssODN at various temperatures at the CAMK2D locus in HEK293T cells**

| **Group Conditions sgRNA** | | **PL1** | | **PL2** | | **PL3** | |
|---|---|---|---|---|---|---|---|
| | | **37°C-37°C-37°C** | | **37°C-32°C-37°C** | | **37°C-32°C-32°C** | |
| | | **CAMK-CR1** | **CAMK-CR2** | **CAMK-CR1** | **CAMK-CR2** | **CAMK-CR1** | **CAMK-CR2** |
| **ssODN input** | **None** | 0.01±0 | 0.02±0 | 0±0 | 0.01±0 | 0±0 | 0±0 |
| | **10 pmol** | 1.12±0.06^{a} | 1.51±0.07^{b} | 7.13±0.21^{a} | 10.45±0.21^{b} | 6.65±0.06^{a} | 10.45±0.31^{b} |
| | **30 pmol** | 0.57±0.02^{c} | 0.81±0.04^{d} | 3.94±0.05^{c} | 6.09±0.19^{d} | 3.40±0.06^{c} | 7.10±0.4^{d} |

| | | | | | | | |
|---|---|---|---|---|---|---|---|
| The experiments were carried out at different temperatures and HDR efficiencies were determined by ddPCR using probes specific for wild type and mutant sequences at the CAMK2D locus. Data presented as mean percentage of mutation containing droplets ± SEM from three replicates. The significance of HDR efficiency difference among three temperature conditions for each gRNA and ssODN treatment were analyzed by one way ANOVA.(a,b,c,d: one way ANOVA P<0.0001, follow-up Dunnett's multiple comparison, PL2 versus PL1: P = 0.0001, PL3 versus PL1: P = 0.0001) | | | | | | | |

**Table 7. 'Cold shock' enhances HDR efficiency at the CAMK2D locus in HEK293T cells as determined by NGS**

| | **sgRNA** | **CAMK-CR1** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.06 | 0.01 | 0.19 | 0.01 | 99.75 | 0.01 |
| **PL2** | **37°C-32°C-37°C** | 0 | 0 | 0.16 | 0.01 | 0.08 | 0.01 | 99.76 | 0.01 |
| **PL3** | **37°C-32°C-32°C** | 0 | 0 | 0.16 | 0 | 0.11 | 0.01 | 99.73 | 0.01 |
| | | | | | | | | | |

| | **sgRNA** | **CAMK-CR1** | | | | | | | |
|---|---|---|---|---|---|---|---|---|---|
| | **ssODN** | **C-CR2** | | | | | | | |
| | **ssODN Input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0.17 | 0.06 | 0.1 | 0.03 | 0.6 | 0.03 | 99.12 | 0.1 |
| **PL2** | **37°C-32°C-37°C** | 1.93 | 0.36 | 0.91 | 0.09 | 3.55 | 0.5 | 93.6 | 0.94 |
| **PL3** | **37°C-32°C-32°C** | 1.61 | 0.28 | 0.72 | 0.06 | 3.07 | 0.53 | 94.59 | 0.87 |
| | | | | | | | | | |

| | **sigRNA** | **CAMK-CR1** | | | | | | | |
|---|---|---|---|---|---|---|---|---|---|
| | **ssODN** | **C-CR2** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | \| StdErr |
| **PL1** | **37°C-37°C-37°C** | 0.09 | 0.02 | 0.02 | 0 | 0.41 | 0.06 | 99.49 | 0.08 |
| **PL2** | **37°C-32°C-37°C** | 1.75 | 0.4 | 0.75 | 0.31 | 4.04 | 2.27 | 93.47 | 2.97 |
| **PL3** | **37°C-32°C-32°C** | 1.46 | 0.48 | 0.52 | 0.32 | 4 | 2.65 | 94.03 | 3.44 |
| | | | | | | | | | |

| | **sgRNA** | **CAMK-CR2** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.01 | 0 | 0.29 | 0.04 | 99.7 | 0.05 |
| **PL2** | **37°C-32°C-37°C** | 0 | 0 | 0.12 | 0.03 | 0.34 | 0.01 | 99.54 | 0.04 |
| **PL3** | **37°C-32°C-32°C** | 0 | 0 | 0.13 | 0.03 | 0.29 | 0.04 | 99.58 | 0.07 |
| | | | | | | | | | |

| | **sgRNA** | **CAMK-CR2** | | | | | | | |
|---|---|---|---|---|---|---|---|---|---|
| | **ssODN** | **C-CR2** | | | | | | | |
| | **ssODN Input** | **10 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0.13 | 0.03 | 0.07 | 0.02 | 0.74 | 0.1 | 99.06 | 0.15 |
| **PL2** | **37°C-32°C-37°C** | 2.83 | 0.56 | 0.89 | 0.27 | 10.44 | 2.99 | 85.85 | 3.81 |
| **PL3** | **37°C-32°C-32°C** | 2.66 | 0.92 | 0.84 | 0.29 | 10.23 | 4.52 | 86.27 | 5.73 |
| | | | | | | | | | |

| | **sgRNA** | **CAMK-CR2** | | | | | | | |
|---|---|---|---|---|---|---|---|---|---|
| | **ssODN** | **C-CR2** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0.19 | 0.03 | 0.02 | 0.02 | 0.44 | 0.02 | 99.36 | 0.04 |
| **PL2** | **37°C-32°C-37°C** | 3.37 | 0.8 | 0.85 | 0.23 | 9.2 | 2.91 | 86.59 | 3.87 |
| **PL3** | **37°C-32°C-32°C** | 3.21 | 1.12 | 0.57 | 0.26 | 7.24 | 4.1 | 88.98 | 5.33 |

### V. "Cold shock' does not affect the expression of pluripotency makers

To test if exposing mc-IPSCs to periods of cold could affect the ability of the cells to be differentiated into various cellular lineages, we performed an identical `cold shock' protocol as was used to determine if the process effected rates of HDR, and stained the cells with antibodies that recognize protein antigens whose expression is indicative of pluripotency. The results of these studies demonstrate that the expression of the markers SSEA3, Nanog and OCT4, do not change as a result of the cells being exposed to either 24 hrs or 48 hrs 32 °C temperature condition (Figure 7a-c).

### VI. 'Cold shock' is effective at increasing the rates of HDR across a range of temperature conditions

To test the effect of exposing cells to temperature conditions lower than 32 °C, on the efficiency of HDR, we repeated the cell culturing protocol previously described but exposed the mc-IPSCs to either 30 °C, 28 °C as well as 32 °C for both 24h and 48hrs.

The resulting PCR amplicons were analyzed by both ddPCR and NGS (Table 8, Table 9, and Figure 8). In general, increases in total HDR (Table 9 and Figure 8) and increases in 'perfect' HDR (Figure 8) were comparable across the three temperatures tested and these data demonstrate that increased HDR is not depended on the 32 °C temperature condition.

**Table 8. HDR efficiencies at various temperatures at the CAMK2D locus in mc-iPSCs as determined by ddPCR**

| **Group Conditions sgRNA** | | **PL1** | **PL2** | **PL3** | **PL4** | **PL5** | **PL6** | **PL7** |
|---|---|---|---|---|---|---|---|---|
| | | **37°C-37°C-37°C** | **37°C-32°C-37°C** | **37°C-32°C-32°C** | **37°C-30°C-37°C** | **37°C-30°C-30°C** | **37°C-28°C-37°C** | **37°C-28°C-28°C** |
| | | **CAMK-CR1** | **CAMK-CR1** | **CAMK-CR1** | **CAMK-CR1** | **CAMK-CR1** | **CAMK-CR1** | **CAMK-CR1** |
| **ssODN input** | **None** | 0.00±0.00 | 0.00±0.00 | 0.00±0.00 | 0.00±0.00 | 0.00±0.00 | 0.00±0.00 | 0.00±0.00 |
| | **30 pmol** | 6.52±0.51 | 17.35±0.38 | 21.6±0.23 | 17.9±0.12 | 23.25±0.61 | 13.7±0.06 | 18.6±0.12 |
| | | | | | | | | |

| **Group Conditions sgRNA** | | **PL1** | **PL2** | **PL3** | **PL4** | **PL5** | **PL6** | **PL7** |
|---|---|---|---|---|---|---|---|---|
| | | **37°C-37°C-37°C** | **37°C-32°C-37°C** | **37°C-32°C-32°C** | **37°C-30°C-37°C** | **37°C-30°C-30°C** | **37°C-28°C-37°C** | **37°C-28°C-28°C** |
| | | **CAMK-CR2** | **CAMK-CR2** | **CAMK-CR2** | **CAMK-CR2** | **CAMK-CR2** | **CAMK-CR2** | **CAMK-CR2** |
| **ssODN input** | **None** | 0.00±0.00 | 0.00±0.00 | 0.00±0.00 | 0.00±0.00 | 0.00±0.00 | 0.00±0.00 | 0.00±0.00 |
| | **30 pmol** | 12.15±0.49 | 29.55±1.13 | 39.95±1.07 | 28.65±0.72 | 38.9±0.29 | 25.45±0.03 | 35.9±0.46 |

| | | | | | | | | |
|---|---|---|---|---|---|---|---|---|
| The experiments were carried out at different temperatures as described in "material and methods" and HDR efficiencies were determined by ddPCR using probes specific for wild type and mutant sequences at the CAMK2D locus. Data presented as mean percentage of droplets containing the mutant allele± standard error from two replicates | | | | | | | | |

**Table 9. 'Cold shock' enhances HDR efficiency at the CAMK2D locus in mc-iPSC cells as determined by NGS**

| | **sgRNA** | **CAMK-CR1** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 0 | 0 | 0.18 | 0.05 | 0.04 | 0.01 | 99.79 | 0.04 |
| **PL2** | **37°C-32°C-37°C** | 0.01 | 0.01 | 0.21 | 0.06 | 0.02 | 0.01 | 99.78 | 0.07 |
| **PL3** | **37°C-32°C-32°C** | 0.01 | 0.01 | 0.12 | 0.01 | 0.05 | 0.03 | 99.83 | 0.04 |
| **PL4** | **37°C-30°C-37°C** | 0 | 0 | 0.07 | 0.01 | 0.01 | 0 | 99.92 | 0.02 |
| **PL5** | **37°C-30°C-30°C** | 0 | 0 | 0.09 | 0.03 | 0.03 | 0.02 | 99.88 | 0.01 |
| **PL6** | **37°C-28°C-28°C** | 0 | 0 | 0.13 | 0.03 | 0.06 | 0.01 | 99.82 | 0.02 |
| **PL7** | **37°C-28°C-28°C** | 0 | 0 | 0.12 | 0.02 | 0.01 | 0 | 99.88 | 0.02 |
| | | | | | | | | | |

| | **sgRNA** | **CAMK-CR1** | | | | | | | |
|---|---|---|---|---|---|---|---|---|---|
| | **ssODN** | **C-CR2** | | | | | | | |
| | **ssODN Input** | **30 pmol** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | 37°C-37°C-37°C | 3.6 | 0.42 | 1.41 | 0.02 | 10.83 | 0.81 | 84.17 | 1.25 |
| **PL2** | **37°C-32°C-37°C** | 8.32 | 0.58 | 3.85 | 0.63 | 18.38 | 2.02 | 69.46 | 3.22 |
| **PL3** | **37°C-32°C-32°C** | 10.65 | 0.2 | 4.45 | 0.19 | 21.38 | 1.38 | 63.54 | 1 |
| **PL4** | **37°C-30°C-37°C** | 8.47 | 0.21 | 3.56 | 0.29 | 16.6 | 1.16 | 71.39 | 0.66 |
| **PL5** | **37°C-30°C-30°C** | 9.74 | 0.06 | 4.49 | 0.15 | 23.19 | 3.58 | 62.59 | 3.67 |
| **PL6** | **37°C-28°C-28°C** | 7.16 | 0.32 | 3.49 | 0.21 | 14.15 | 0.38 | 75.2 | 0.15 |
| **PL7** | **37°C-28°C-28°C** | 8.79 | 0.29 | 4.19 | 0.12 | 15.12 | 0.67 | 71.91 | 1.07 |

| | **sgRNA** | **CAMK-CR2** | | | | | | | |
|---|---|---|---|---|---|---|---|---|---|
| | **ssODN** | **None** | | | | | | | |
| | **ssODN Input** | **None** | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | \| StdErr |
| **PL1** | **37°C-37°C-37°C** | 0 | 0 | 0.21 | 0.03 | 0.24 | 0.02 | 99.56 | 0.04 |
| **PL2** | **37°C-32°C-37°C** | 0 | 0 | 0.23 | 0.04 | 0.16 | 0.01 | 99.61 | 0.02 |
| **PL3** | **37°C-32°C-32°C** | 0 | 0 | 0.65 | 0.07 | 0.32 | 0.11 | 99.03 | 0.04 |
| **PL4** | **37°C-30°C-37°C** | 0 | 0 | 0.66 | 0.01 | 0.22 | 0 | 99.13 | 0 |
| **PL5** | **37°C-30°C-30°C** | 0 | 0 | 1.07 | 0.37 | 0.47 | 0.17 | 98.47 | 0.21 |
| **PL6** | **37°C-28°C-28°C** | 0 | 0 | 0.69 | 0.02 | 0.4 | 0.05 | 98.92 | 0.07 |
| **PL7** | **37°C-28°C-28°C** | 0.01 | 0.01 | 1.37 | 0.03 | 0.35 | 0.11 | 98.28 | 0.14 |
| | | | | | | | | | |

| | **sgRNA** | **CAMK-CR2** | | | | | | | |
|---|---|---|---|---|---|---|---|---|---|
| | **ssODN** | C-CR2 | | | | | | | |
| | **ssODN Input** | **30** pmol | | | | | | | |
| **Group** | **Temperature** | (PctPerfectOligo) | | (PctEditedOligo) | | (PctPartialOligo) | | (PctNonOligo) | |
| | | Average | StdErr | Average | StdErr | Average | StdErr | Average | StdErr |
| **PL1** | **37°C-37°C-37°C** | 6.35 | 0.13 | 1.5 | 0.02 | 13.87 | 0.32 | 78.29 | 0.42 |
| **PL2** | **37°C-32°C-37°C** | 15.05 | 0.29 | 2.58 | 0.14 | 24.1 | 1.43 | 58.29 | 1.85 |
| **PL3** | **37°C-32°C-32°C** | 20.37 | 3.1 | 3.24 | 0.45 | 25.99 | 1.14 | 50.41 | 2.41 |
| **PL4** | **37°C-30°C-37°C** | 13.1 | 0.83 | 2.56 | 0.03 | 24.89 | 0.37 | 59.45 | 1.24 |
| **PL5** | **37°C-30°C-30°C** | 17.55 | 0.52 | 3.18 | 0.03 | 28.79 | 0.34 | 50.49 | 0.82 |
| **PL6** | **37°C-28°C-28°C** | 11.27 | 0.56 | 2.71 | 0.23 | 23.38 | 0.14 | 62.65 | 0.64 |
| **PL7** | **37°C-28°C-28°C** | 14.58 | 0.78 | 2.49 | 0.01 | 26 | 0.63 | 56.94 | 1.41 |

### Discussion

The rapid development of CRISPR-based genome engineering methodologies requires an agnostic and systematic process of evaluation in order to gain the maximum benefit from this technology. Here we report an optimized CRISPR modality/delivery combination that is highly effective in promoting HDR in the mc-iPSC line. We then used this method to evaluate if exposure to lower temperature can increase the efficiency of HDR and found that exposure 32 °C or `cold shock' for 24 or 48 hours is capable of increasing rates of HDR 2 fold or more. Given that appreciable effort is being exerted to find ways of increasing rates of HDR, including 'driving' the repair process away from non-homologous end joining toward HDR with chemical inhibition of DNA repair enzymes (16) and blocking and synchronizing cells at the G2/M boundary with other inhibitors (13), our method provides a more 'physiological' approach that might have broader application, especially where gene editing is being applied in a therapeutic setting.

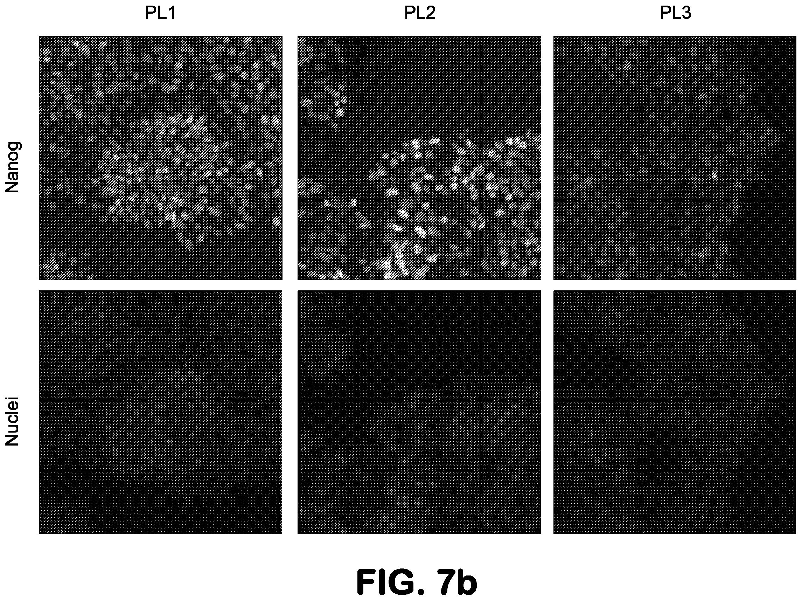

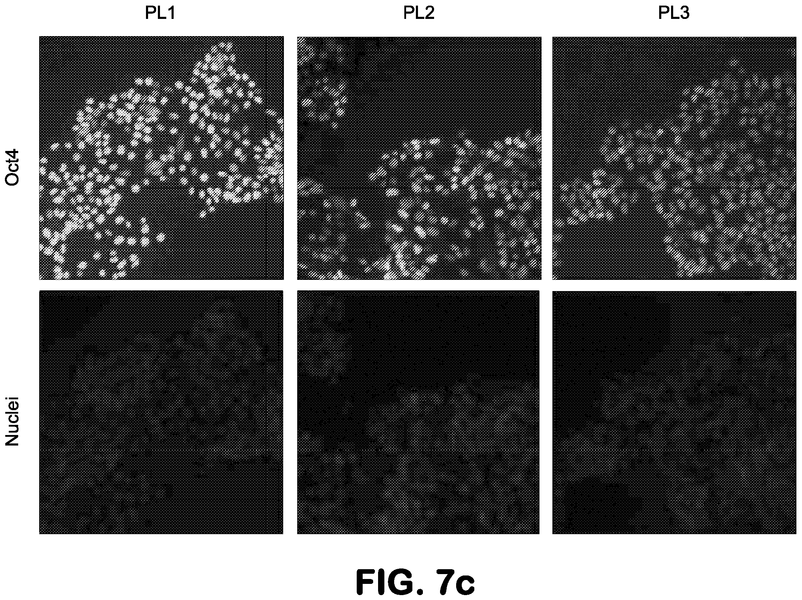

Interestingly, the `cold shock' effect is more dramatic when lower HDR rates are observed (1-20% of the alleles) and diminishes as the base HDR rate increases above 30% or more. This suggests a theoretical limit to the number of alleles that can be altered, at least with this approach. The exact mechanism by which the `cold shock' increases HDR is currently under investigation. A mechanism similar to how zinc finger nucleases increase indel formation may be plausible (17). We observe that with very efficient CRISPRs the effect of the `cold shock' on indel formation is minimal, as observed with CAMK2D guide CAMK-CR1 and TGFBR1 guide TR-CR2. Conversely, when cutting efficiencies and indel formation are lower, as that observed at the CAMK2D locus in HEK293 cells, the increases in cutting efficiency and indel formation are more pronounced. Increased indel formation can clearly contribute to higher HDR rates but does not account for all the increase observed with `cold shock' or explain why `perfect HDR' is favored under cold conditions. A possible mechanism that may contribute to increased HDR rates is that growing cells at 32 °C impacts the cell cycle, with more cells accumulating in G2/M; however, our initial observations to date do not show any cell cycle effects to support this hypothesis (data not shown). A third and more likely contributing factor is that the cold has a thermodynamic effect that acts to stabilize recombination intermediates. Work is currently ongoing to understand the mechanism in detail. One potential concern is that the `cold shock' might adversely affect pluripotency. Preliminary analysis looking at three standard markers of pluripotency, Oct4, SSEA3, and Nanog (18,19) suggest this is not an issue, although some loss of Nanog expression may occur with prolonged exposure to cold (Figure 7). Clearly more work is needed to ensure that `cold shock' is effective and generalizable across cell lines and applications.

We also show that the structure of the donor oligo used to promote HDR can have profound effects on both the overall frequency and type of HDR that takes place. Across the two loci tested, both oligo designs, symmetrical target strand and asymmetrical non-target strand, could produce total HDR to high levels however the symmetric, target strand oligos produced `perfect HDR' more efficiently then asymmetric non-target strand oligos especially under conditions of `cold shock'. While these data are in disagreement with that of Richardson et al. (14), our data do agree with those of Paquet et al., whose donor oligos were designed to the same strand (15).

In summary, we have developed a protocol for performing gene editing in iPSCs that does not require the use of nucleofection or selection to obtain a population of cells that have efficiently undergone directed genomic sequence alteration by the process of HDR. We have also shown that HDR can be effectively increased by the incorporation of a simple, brief, and physiological exposure to a lower temperature which will have broad utility across many genome engineering application.

### REFERENCES

1 Hockemeyer, D. & Jaenisch, R. Induced Pluripotent Stem Cells Meet Genome Editing. Cell stem cell 18, 573-586, (2016).
2 Jang, Y. Y. & Ye, Z. Gene correction in patient-specific iPSCs for therapy development and disease modeling. Human genetics 135, 1041-1058, (2016).
3 Bojesen, S. E. et al. Multiple independent variants at the TERT locus are associated with telomere length and risks of breast and ovarian cancer. Nat Genet 45, 371-384, (2013).
4 Chiba, K. et al. Cancer-associated TERT promoter mutations abrogate telomerase silencing. eLife 4, e07918, (2015).
5 Mali, P. et al. RNA-Guided Human Genome Engineering via Cas9. Science (New York, N.Y.) 339, 823-826, (2013).
6 Chen, F. et al. High-frequency genome editing using ssDNA oligonucleotides with zinc-finger nucleases. Nat Meth 8, 753-755, (2011).
7 Soldner, F. et al. Generation of Isogenic Pluripotent Stem Cells Differing Exclusively at Two Early Onset Parkinson Point Mutations. Cell 146, 318-331, (2011).
8 Miyaoka, Y. et al. Systematic quantification of HDR and NHEJ reveals effects of locus, nuclease, and cell type on genome-editing. Scientific reports 6, 23549, (2016).
9 Zhang, Y. et al. Generation of a human induced pluripotent stem cell line via CRISPR-Cas9 mediated integration of a site-specific homozygous mutation in CHMP2B. Stem cell research 17, 151-153, (2016).
10 Yusa, K. et al. Targeted gene correction of alpha1-antitrypsin deficiency in induced pluripotent stem cells. Nature 478, 391-394, (2011).
11 Yang, L. et al. Optimization of scarless human stem cell genome editing. Nucleic acids research 41, 9049-9061, (2013).
12 Miyaoka, Y. et al. Isolation of single-base genome-edited human iPS cells without antibiotic selection. Nature methods 11, 291-293, (2014).
13 Lin, S., Staahl, B. T., Alla, R. K. & Doudna, J. A. Enhanced homology-directed human genome engineering by controlled timing of CRISPR/Cas9 delivery. Elife 3, e04766, (2014).
14 Richardson, C. D., Ray, G. J., DeWitt, M. A., Curie, G. L. & Corn, J. E. Enhancing homology-directed genome editing by catalytically active and inactive CRISPR-Cas9 using asymmetric donor DNA. Nat Biotech advance online publication, doi:10.1038/nbt.3481, (2016).
15 Paquet, D. et al. Efficient introduction of specific homozygous and heterozygous mutations using CRISPR/Cas9. Nature 533, 125-129, (2016).
16 Maruyama, T. et al. Increasing the efficiency of precise genome editing with CRISPR-Cas9 by inhibition of nonhomologous end joining. Nature biotechnology 33, 538-542, (2015).
17 Doyon, Y. et al. Transient cold shock enhances zinc-finger nuclease-mediated gene disruption. Nat Meth 7, 459-460, (2010).
18 Yu, J. et al. Induced Pluripotent Stem Cell Lines Derived from Human Somatic Cells. Science (New York, N.Y.) 318, 1917-1920, (2007).
19 Takahashi, K. et al. Induction of pluripotent stem cells from adult human fibroblasts by defined factors. Cell 131, 861-872, (2007).

## Claims

1. A method for increasing the efficiency of homology directed repair (HDR) in the genome of a cell, comprising:
(a) introducing into the cell: (i) a nuclease; and (ii) a donor nucleic acid which comprises a modification sequence to be inserted into the genome, wherein the donor nucleic acid contains symmetrical homology arms and is complementary to the DNA strand in the genome which is cleaved by the nuclease; and
(b) subjecting the cell to a temperature shift from 37 °C to a lower temperature which is between 28 °C and 35 °C;
wherein the nuclease cleaves the genome at a cleavage site in the cell, and the donor nucleic acid directs the repair of the genome sequence with the modification sequence through an increased rate of HDR;
wherein the method does not encompass a process for modifying the germ line genetic identity of human beings.

2. The method of claim 1, wherein the lower temperature is between 30 °C and 33 °C.

3. The method of claim 1, wherein the cell is grown at the lower temperature for at least 24 hours.

4. The method of claim 1, wherein the cell is a mammalian cell.

5. The method of claim 4, wherein the cell is selected from a stem cell, an induced pluripotent stem cell (iPSC), or a primary cell.

6. The method of claim 1, wherein the nuclease is a CRISPR nuclease selected from a Cas nuclease or a Cpf1 nuclease.

7. The method of claim 6, wherein the nuclease is a Cas9 nuclease.

8. The method of claim 6, wherein the CRISPR nuclease is introduced into the cell along with a sgRNA either in a DNA format or an RNA format.

9. The method of claim 8, wherein the sgRNA is synthetic and chemically modified.

10. The method of claim 8, wherein a DNA encoding the Cas9 nuclease and a sgRNA is introduced into the cell.

11. The method of claim 8, wherein a sgRNA/Cas9 RNP is introduced into the cell.

12. The method of claim 8, wherein sgRNA/Cas9 mRNAs are introduced into the cell.

13. The method of claim 1, wherein the rate of homology directed repair (HDR) is increased by at least 1.5 fold.

14. A cell for use in a therapeutic method of providing a protein of interest to a subject in need thereof, comprising:
(a) introducing a donor nucleic acid encoding a protein of interest into the cell according to the method of claim 1; and
(b) introducing the cell into a subject, such that the protein of interest is expressed in the subject;
wherein the method does not encompass a process for modifying the germ line genetic identity of human beings.

## Patentansprüche

1. Verfahren zur Steigerung der Effizienz von Homologie-gesteuerter Reparatur (HDR) im Genom einer Zelle, umfassend:
(a) Einbringen in die Zelle: (i) einer Nuklease; und (ii) einer Donor-Nukleinsäure, die eine in das Genom einzufügende Modifikationssequenz umfasst, wobei die Donor-Nukleinsäure symmetrische Homologiearme enthält und zum DNA-Strang im Genom, dessen Spaltung durch die Nuklease erfolgt, komplementär ist; und
(b) Aussetzen, der Zelle, einer Temperaturverlagerung von 37 °C auf eine niedrigere Temperatur, die zwischen 28 °C und 35 °C ist;
wobei die Nuklease das Genom an einer Spaltungsstelle in der Zelle spaltet, und die Donor-Nukleinsäure die Reparatur der Genomsequenz mit der Modifikationssequenz durch eine gesteigerte HDR-Rate steuert;
wobei das Verfahren keinen Vorgang zum Modifizieren der genetischen Identität der Keimbahn menschlicher Lebewesen einbegreift.

2. Verfahren nach Anspruch 1, wobei die niedrigere Temperatur zwischen 30 °C und 33 °C ist.

3. Verfahren nach Anspruch 1, wobei die Zelle bei der niedrigeren Temperatur für mindestens 24 Stunden gezüchtet wird.

4. Verfahren nach Anspruch 1, wobei die Zelle eine Säugerzelle ist.

5. Verfahren nach Anspruch 4, wobei die Zelle aus einer Stammzelle, einer induzierten pluripotenten Stammzelle (iPSC) oder einer Primärzelle ausgewählt ist.

6. Verfahren nach Anspruch 1, wobei die Nuklease eine aus einer Cas-Nuklease oder einer Cpf1-Nuklease ausgewählte CRISPR-Nuklease ist.

7. Verfahren nach Anspruch 6, wobei die Nuklease eine Cas9-Nuklease ist.

8. Verfahren nach Anspruch 6, wobei die CRISPR-Nuklease in die Zelle gemeinsam mit einer sgRNA entweder in einem DNA-Format oder in einem RNA-Format eingebracht wird.

9. Verfahren nach Anspruch 8, wobei die sgRNA synthetisch ist und chemisch modifiziert ist.

10. Verfahren nach Anspruch 8, wobei eine DNA, codierend für die Cas9-Nuklease und eine sgRNA, in die Zelle eingebracht wird.

11. Verfahren nach Anspruch 8, wobei ein sgRNA/Cas9-RNP in die Zelle eingebracht wird.

12. Verfahren nach Anspruch 8, wobei sgRNA/Cas9-mRNAs in die Zelle eingebracht werden.

13. Verfahren nach Anspruch 1, wobei die Rate von Homologie-gesteuerter Reparatur (HDR) um mindestens das 1,5-Fache gesteigert wird.

14. Zelle zur Verwendung in einem therapeutischen Verfahren zur Bereitstellung eines Proteins von Interesse für ein Individuum mit Bedarf daran, umfassend:
(a) Einbringen einer Donor-Nukleinsäure, codierend für ein Protein von Interesse, in die Zelle gemäß dem Verfahren aus Anspruch 1; und
(b) Einbringen der Zelle in ein Individuum auf solche Art, dass das Protein von Interesse im Individuum exprimiert wird;
wobei das Verfahren keinen Vorgang zum Modifizieren der genetischen Identität der Keimbahn menschlicher Lebewesen einbegreift.

## Revendications

1. Procédé pour augmenter l'efficacité de réparation par recombinaison homologue (HDR) dans le génome d'une cellule, comprenant :
(a) l'introduction dans la cellule : (i) d'une nucléase, et (ii) d'un acide nucléique donneur qui comprend une séquence de modification à insérer dans le génome, dans lequel l'acide nucléique donneur contient des bras symétriques d'homologie et est complémentaire au brin d'ADN dans le génome qui est clivé par la nucléase, et
(b) la soumission de la cellule à un changement de température de 37 °C à une température inférieure qui est entre 28 °C et 35 °C,
dans lequel la nucléase clive le génome au niveau d'un site de clivage dans la cellule, et l'acide nucléique donneur dirige la réparation de la séquence de génome avec la séquence de modification grâce à un taux accru de HDR,
dans lequel le procédé n'englobe pas un processus de modification de l'identité génétique germinale des êtres humains.

2. Procédé selon la revendication 1, dans lequel la température inférieure est comprise entre 30 °C et 33 °C.

3. Procédé selon la revendication 1, dans lequel la cellule est cultivée à la température inférieure pendant au moins 24 heures.

4. Procédé selon la revendication 1, dans lequel la cellule est une cellule de mammifère.

5. Procédé selon la revendication 4, dans lequel la cellule est sélectionnée parmi une cellule souche, une cellule souche pluripotente induite (iPSC) ou une cellule primaire.

6. Procédé selon la revendication 1, dans lequel la nucléase est une nucléase CRISPR sélectionnée parmi une nucléase Cas ou une nucléase Cpf1.

7. Procédé selon la revendication 6, dans lequel la nucléase est une nucléase Cas9.

8. Procédé selon la revendication 6, dans lequel la nucléase CRISPR est introduite dans la cellule en même temps qu'un sgARN dans un format d'ADN ou dans un format d'ARN.

9. Procédé selon la revendication 8, dans lequel le sgARN est synthétique et chimiquement modifié.

10. Procédé selon la revendication 8, dans lequel un ADN codant pour la nucléase Cas9 et un sgARN est introduit dans la cellule.

11. Procédé selon la revendication 8, dans lequel une RNP sgARN/Cas9 est introduite dans la cellule.

12. Procédé selon la revendication 8, dans lequel des ARNm sgARN/Cas9 sont introduits dans la cellule.

13. Procédé selon la revendication 1, dans lequel le taux de réparation par recombinaison homologue (HDR) est augmenté d'au moins un facteur 1,5.

14. Cellule pour utilisation dans un procédé thérapeutique pour fournir une protéine d'intérêt à un sujet en ayant besoin, comprenant :
(a) l'introduction d'un acide nucléique donneur codant pour une protéine d'intérêt dans la cellule selon le procédé de la revendication 1 ; et
(b) l'introduction de la cellule chez un sujet, de façon à ce que la protéine d'intérêt soit exprimée chez le sujet ;
où le procédé n'englobe pas un processus de modification de l'identité génétique germinale des êtres humains.
